# EUROPÄISCHE PATENTSCHRIFT

(11) **EP 2 990 900 B1**
(45) Veröffentlichungstag und Bekanntmachung des Hinweises auf die Patenterteilung: **12.04.2017**
(21) Anmeldenummer: 14002995.0
(22) Anmeldetag: 29.08.2014
(51) Int. Cl.: G05B 23/02

(54) **System und Verfahren zur Steuerung, Erfassung, Regelung und/oder Analyse von biologischen, biochemischen, chemischen und/oder physikalischen Prozessen durch Datenkorrelation**
System and method for control, acquisition, configuration and/or analysis of biological, biochemical, chemical and/or physical processes by using data correlation
Système et méthode pour le contrôle, l'acquisition, la configuration et/ou l'analyse des processus biologiques, biochimiques, chimiques et/ou physiques en utilisant une corrélation de données

(43) Veröffentlichungstag der Anmeldung: 02.03.2016
(73) Patentinhaber: Sartorius Stedim Biotech GmbH, 37079 Göttingen (DE)
(72) Erfinder: Weggler, Sophie, 37073 Göttingen (DE); Götje, Nils, 30161 Hannover (DE); Krumbein, Sascha, 37214 Witzenausen (DE)
(74) Vertreter: Müller-Boré & Partner Patentanwälte PartG mbB

(56) Entgegenhaltungen:
- WO-A1-2014/006807
- US-A1- 2003 028 269
- US-A1- 2005 197 805
- US-B1- 6 546 134
- US-B1- 6 727 096

## Beschreibung

Die vorliegende Erfindung betrifft ein Computersystem, ein computer-implementiertes Verfahren, ein Computerprogrammprodukt sowie eine Benutzerschnittstelle zur Steuerung, Erfassung, Regelung und/oder Analyse von biologischen, biochemischen, chemischen und/oder physikalischen Prozessen.

Beispielsweise ist aus der US 2005/0197805 A1 ein System zur visuellen Darstellung von Daten bekannt, die Geräten einer Prozessanlage entsprechen. Das System ist in der Lage ein Bild anzuzeigen, das für die Geräte und ihre Funktion innerhalb der Prozessanlage repräsentativ ist. Ferner werden Daten angezeigt, die auf Signalverarbeitungsdaten eines oder mehrerer der Geräte basieren.

US 6 727 096 B1 offenbart Computerprogramme und Verfahren zur Überwachung der Abläufe und Eigenschaften parallel verlaufender chemischer Reaktionen.

Bekannte Systeme ermöglichen eine Anzeige von Messdaten in Echtzeit, die über einen Sensor einer Einheit erfasst werden, d.h. die Messdaten, welche angezeigt werden, sind an eine aktuelle Prozesszeit gekoppelt. Eine Anzeige von Messdaten unabhängig von einer absoluten Zeit ihrer Erfassung ist nicht möglich. Die Anzeige erfolgt über eine Anzeigeeinheit, welche an einen Bioreaktor bzw. eine Filtrationsanlage bzw. ein Einfrier-und Auftau-System gekoppelt ist. Ferner ist es konventionell möglich, Parameter, die für die Durchführung eines biologischen, biochemischen, chemischen und/oder physikalischen Prozesses bedeutsam sind, über die Anzeigeeinheit einzustellen. Bei den Parametern kann es sich zum Beispiel um einen pH-Wert, eine Sauerstoffzufuhr, etc. handeln. Dadurch wird eine prozessnahe Visualisierung von Messdaten sowie eine Steuerung von Prozessparametern ermöglicht.

Allerdings ist es bei einer Durchführung von biologischen, biochemischen, chemischen und/oder physikalischen Prozessen in einer Einheit, welche einen Reaktor beinhaltet, von wesentlicher Bedeutung, einen genauen Überblick über wichtige, den Prozess beeinflussende Faktoren sowie eine Auswirkung einer Änderung dieser Faktoren auf den Prozesserfolg zu erhalten. Aus diesem Grund ist es notwendig, einen Verlauf von Messdaten, die z.B. innerhalb ein oder mehrerer Batch-Phasen bzw. Batches unterschiedlicher Prozesse, unterschiedlicher Prozessausgestaltungen und/oder von gleichen und/oder unterschiedlich ausgestalteten Prozessen, die zu verschiedenen absoluten Zeitpunkten gestartet wurden, miteinander zu vergleichen. Konventionelle Systeme ermöglichen es nicht, solch einen Messdatenverlauf in einer zeitlichen Korrelation derart darzustellen, um Aufschlüsse über einen Zusammenhang der erfassten Messdaten zu ermöglichen.

Aufgabe der vorliegenden Erfindung ist es demnach, Messdaten von biologischen, biochemischen, chemischen und/oder physikalische Prozesse in verbesserter Weise zu erfassen und weiterverarbeiten zu können.

Diese Aufgabe wird erfindungsgemäß durch die Merkmale der unabhängigen Ansprüche gelöst. Bevorzugte Ausführungsformen sind Gegenstand der abhängigen Ansprüche.

Gemäß eines ersten Aspekts der Erfindung wird das Computersystem zur Steuerung, Erfassung, Regelung und/oder Analyse von biologischen, biochemischen, chemischen und/oder physikalischen Prozessen gemäß Anspruch 1 bereitgestellt, welches umfasst:
zumindest zwei Einheiten bzw. Units, welche eingerichtet sind, einen Stoff bzw. eine Materie aufzunehmen, um an diesem Stoff zumindest einen biologischen, biochemischen, chemischen und/oder physikalischen Prozess durchzuführen;
wobei jede der Einheiten zumindest einen Sensor aufweist, welcher eingerichtet ist, Messdaten betreffend den Prozess zu erfassen; und
zumindest eine Anzeigeeinheit, über welche die Messdaten der zwei Einheiten jeweils in einer zeitlichen Korrelation dargestellt werden, welche Aufschlüsse über einen Zusammenhang der dargestellten Messdaten ermöglicht.

Ein Reaktor kann z.B. ein Bioreaktor und/oder ein chemischer Reaktor sein, welcher eingerichtet ist, einen Stoff bzw. eine Materie aufzunehmen, an der zumindest ein biologischer, biochemischer, chemischer und/oder physikalischer Prozess durchgeführt werden kann. Bei den Bioreaktoren können sowohl single-use Reaktoren in Form von Beuteln oder Standgeräten als auch feste Behälter aus Edelstahl zum Einsatz kommen. Je nach Art der Kultivierung sind die Bioreaktoren mit festen Materialen zur Ansiedelung adhärenter Zellen gepackt oder als Rührreaktor ausgestaltet.

Alternativ oder zusätzlich zum Reaktor kann eine Einheit insbesondere zumindest einen Filter beinhalten, welcher zur Filtration eines Mediums verwendet werden kann. Beispielsweise kann es sich dabei um einen Filter zur Cross-Flow-Filtration handeln, welcher z.B. zur Reinigung und/oder Abtrennung von Proteinen oder Proteinbestandteilen verwendet werden kann; einen Filter zur Durchführung einer Membranchromatographie, z.B. zur Abtrennung von Zellbestandteilen, Nukleinsäuren, Viren, Mycoplasmen oder Endotoxinen; einen Clarification-Filter, z.B. zur Aufreinigung von Serum- bzw. Plasmalösung; einen Virenfilter, z.B. zur Abtrennung von Viren und/oder Virenbestandteilen; ein UV-C Inaktivierungssystem, z.B. zur Abtrennung von Viren und/oder Virenbestandteilen; und/oder einen Tangential-Flow-Filter bzw. Querstromfilter zur Filtration von Flüssigkeiten, beispielsweise in der Lebensmittel-und/oder Pharmaindustrie.

Alternativ oder zusätzlich zum Reaktor bzw. Filter kann eine Einheit insbesondere zumindest ein Einfrier-und Auftau-System beinhalten, welches beispielsweise eingerichtet ist, bio-pharmazeutische Flüssigkeiten in einer Herstellung und Prozessentwicklung reproduzierbar einzufrieren und aufzutauen.

Bei dem Stoff kann es sich um eine Flüssigkeit, ein Gas und/oder einen Feststoff handeln. Der Stoff kann sich aus zumindest einer Komponente zusammensetzen, welche benötigt wird, um die Durchführung des Prozesses zu ermöglichen. So ist es beispielsweise zur Kultivierung von Mikroorganismen notwendig, wesentliche Bedingungen, die ein optimales Wachstum der Organismen gewährleisten, zu schaffen und aufrechtzuerhalten. Beispielsweise kann es sich bei solchen wichtigen Bedingungen um eine Zusammensetzung eines Nährmediums oder einer Nährlösung t, eines Sauerstoffgehaltes, eines pH-Wertes und/oder einer Temperatur im Bioreaktor handeln. Zweck der Kultivierung kann letztendlich die Gewinnung der Organismen oder Teilen der Organsimen und/oder deren sezernierten oder unsezernierten Stoffwechselprodukte, sein, welche als Wirkstoff in der pharmazeutischen Industrie oder als Grundchemikalie in der chemischen Industrie verwendet werden können. Ferner kann es sich beim Zweck der Kultivierung um einen Abbau chemischer Verbindungen, z.B. in Kläranlagen oder zur Fermentation von Lebensmittels handeln.

Jede Einheit kann dabei individuell aus zumindest einem Reaktor, zumindest einem Sensor sowie optional ein oder mehreren Einzelsteuereinheiten und Prozessgeräten zusammengesetzt werden, die in ihrer Gemeinsamkeit aus logischer Sicht einen Teil eines Werkes darstellen, in welchem ein oder mehrere Prozessaktivitäten durchgeführt werden können. Jede Einheit generiert zu einem gegeben Zeitraum während eines Prozesses immer nur einen Batch. Somit kann mit jeder Einheit ein spezifischer Prozess abgebildet werden, so dass alle zu einer Prozessaufzeichnung bzw. Prozessüberwachung benötigten Messdaten mithilfe des Sensors erfasst und gleichzeitig angezeigt werden können.

Messdaten von den in den Einheiten vorhandenen Stoffen und/oder darin ablaufenden Prozessen, welche mit dem Computersystem erfasst, dargestellt und gegebenenfalls mit Messdaten anderer Einheiten korreliert werden können, unterliegen keinen besonderen Einschränkungen und sind im hohen Maße von der jeweiligen Anwendung und deren Aufgabe oder Fragestellung abhängig. Sie können beispielsweise Zelldichte, Wachstumsrate der Zellen, Menge eines Kulturmediums, Schaumentwicklung des Kulturmediums, pH-Wert des Kulturmediums, Temperatur des Kulturmediums, Sauerstoffgehalt des Kulturmediums, Kohlendioxidgehalt des Kulturmediums, Trübung des Kulturmediums, Kapazität und/oder Leitfähigkeit des Kulturmediums, Konzentration bestimmter Inhalts- oder Nährstoffe des Kulturmediums, Konzentration hergestellter und sezernierter Stoffwechselprodukte, Peptide und/oder Proteine im Kulturmedium, Flussraten in bestimmten Teilen des Bioreaktors, oder Betriebsparameter des Bioreaktors betreffen. Entsprechende Sensoren zur Erfassung solcher oder anderer Messdaten unterliegen keinen besonderen Einschränkungen und sind im Stand der Technik bekannt. Messdaten können in einem spezifischen Zeitintervall erfasst werden. Vorzugsweise kann das Zeitintervall in einem Bereich zwischen etwa 500 bis etwa 60.000 ms liegen. Vorzugsweise können die Messdaten von jeder Einheit von dem Sensor abgerufen werden (Polling).

Im Beispiel einer durchzuführenden Fermentation kann sich eine Einheit aus einem Behälter (z.B. einem Bioreaktor, eine Filtrationsanlage oder ein Einfrier- und Auftausystem), einem Sensor (z.B. einem Gas-Analysegerät zur Analyse von O₂ und/oder CO₂) und einer weiteren Einzelsteuereinheit (z.B. einer Waage) zusammensetzen. Jede dieser Komponenten kann über ein entsprechendes Device an die Einheit gekoppelt sein. Jedes Device kann eine Kommunikationsschnittstelle bzw. Eingabe-/Ausgabe-Schnittstelle bzw. Communication Interface sein. Die Trennung zwischen Einheiten und Devices hat den Vorteil, dass eine Konfiguration einer Einheit, die sich aus zumindest einem Reaktor, zumindest einem Sensor sowie verschiedensten Einzelsteuereinheiten zusammensetzen kann, welche auf einfache und effiziente Weise mithilfe der Devices an eine Einheit, z.B. über ein Netzwerk, angebunden werden können, effizient und einfach realisieren lässt. Außerdem ist eine saubere Trennung zwischen Kommunikationssicht, technischer Sicht und Prozesssicht ermöglicht. So kann im vorangehenden Beispiel das Gas-Analysegerät über eine Universal Serial Bus (USB)-Verbindung an die Einheit gekoppelt werden, wobei ein Sensor Device als Kommunikationsschnittstelle dient. Der Bioreaktor kann beispielsweise via Ethernet Daten mit der Einheit austauschen, wobei ein Reaktor-Device als Kommunikationsschnittstelle dienen kann. Eine Waage kann über ein Einzelsteuereinheit-Device an die Einheit gekoppelt werden. Optional können benötigte interne Einzelsteuereinheiten an die Einheit gekoppelt werden. Beispielsweise kann ein Berechnungs-Modul eingesetzt werden, welches anstelle eines Devices zyklische Berechnungen durchführt und damit ähnlich wie ein Device zyklisch das Ergebnis als Wert an ein Control Module liefert. Ein Sample Data Management Modul kann die manuelle Eingabe von entnommenen Probedaten ermöglichen, welche im Labor erst aus einer Probe ermittelt wurden. Diese logische Zusammenfassung der jeweiligen Komponenten zu einer Einheit hat den Vorteil, dass eine Trennung zwischen der Gerätekommunikation (Devices) und einem Monitoring, also einer Prozessebene (Einheiten), erzielt wird.

Jede Einheit weist zumindest einen Sensor auf, welcher eingerichtet ist, spezifische, d.h. vorbestimmte bzw. vorbestimmbare, Messdaten bezüglich des Prozesses zu erfassen. Beispielsweise kann es sich dabei um einen Gassensor, z.B. einen O₂- bzw. CO₂-Sauerstoffsensoᵣ, einen pH-Sensor, einen Glucose-Sensor, einen Lactat-Sensor sowie jeden anderen Sensor, der eingerichtet ist, eine geeignete Messgröße in Hinblick auf den durchzuführenden Prozess zu erfassen, oder auch um eine beliebige Kombination der Sensoren handeln. Optional kann jede Einheit zusätzlich zumindest eine Einzelsteuereinheit aufweisen. Entsprechendes gilt für Filtrationsanlagen. Die Trennung Unit/Devices dient dem modularen Aufbau mit dem sich jeder Benutzer seinen eigenen Prozess standardisiert zusammenkonfigurieren kann, ohne Engineering zu betreiben.

Die Messdaten können über die Anzeigeeinheit, beispielsweise in Form von Messkurven, dargestellt werden. Alternativ dazu oder zusätzlich können die Messdaten in Form von ein oder mehreren Diagrammen, z.B. Kreisdiagrammen, Liniendiagrammen, Balkendiagrammen, Organigrammen, Gantt-Diagrammen, Fluss-Diagrammen, Ursache-Wirkungs-Diagrammen, Blockdiagrammen etc., dargestellt werden. Die Messdaten können z.B. vom Typ Float, Integer, Double, String oder Boolean sein.

Die Messdaten können in einer Datenbank gespeichert werden, die geeignet ist, die gespeicherten Messdaten schnell und in kleinen zeitlichen Abständen auf einen entsprechenden Datenbankaufruf hin auszugeben. Beispielsweise kann in der Datenbank eine effiziente Datenkomprimierung implementiert sein.

Beispielsweise kann sich ein Messdatum aus folgenden Variablen zusammensetzen:
- MappingShortID → Identifizierung Kontrollmodul-Variable;
- Timestamp → Zeitstempel: Wann ist das Messdatum in der Einheit eingegangen?;
- Value → Messwert des Messdatums;
- Quality → binärer Wert, z.B. 0 für "good", 1 für "bad"; die Qualität kann ausschlaggebend dafür sein, ob das Messdatum über die Anzeigeeinheit angezeigt wird;
- Checksum bzw. Prüfsumme → Wird vom Computersystem generiert, um eine Datenmanipulation zu verhindern; dies hat den Vorteil, dass die Sicherheit im Computersystem erhöht wird.

Um den Prozess der Kultivierung der Zellen zu optimieren, ist es wesentlich, einen genauen Überblick über wichtige Faktoren des Prozesses einerseits, z.B. einen Sauerstoffgehalt, sowie eine Auswirkung einer Änderung dieser Faktoren auf den Prozesserfolg andererseits, z.B. eine Auswirkung auf das Zellwachstum durch eine Sauerstoffzufuhr bzw. durch eine Zugabe eines neuen Nährmediums, zu erhalten. Dies kann auf effiziente Weise, z.B. bei zwei zum selben Zeitpunkt laufenden Prozessen in unterschiedlichen Einheiten, realisiert werden, indem Messdaten der Sensoren der zwei Einheiten jeweils in einer zeitlichen Korrelation dargestellt werden, welche Aufschlüsse über einen Zusammenhang der dargestellten Messdaten ermöglicht.

Dies hat insbesondere den Vorteil, dass Auswirkungen unterschiedlicher wichtiger Prozessfaktoren bzw. einer Änderung ein oder mehrerer solcher Prozessfaktoren in Echtzeit auf eine einfache und effiziente Weise erfasst werden können. Somit wird eine kontinuierliche Optimierung bzw. Verbesserung der Prozessabläufe ermöglicht. Insbesondere wird dadurch eine verbesserte, kontinuierliche Benutzer-Maschinen Interaktion erzielt, da eine Einstellung von biologischen, chemischen, biochemischen und/ oder physikalischen Prozessparametern während laufender Prozesse in den Einheiten eingestellt werden kann. Dies kann insbesondere in Abhängigkeit durch eine Eingabe eines Benutzers und/oder automatisch durch eine automatische Erkennung der zeitlichen Korrelation der Messdaten erfolgen. Dadurch wird eine Koordinierung bzw. Abstimmung der technischen Abläufe in den jeweiligen Einheiten ermöglicht.

Darüber hinaus wird über die Anzeigeeinheit eine Anzeige unterschiedlicher technischer Zustände, die in den jeweiligen Einheiten herrschen, ermöglicht.

Insbesondere kann über die Anzeigeeinheit ein Alarm angezeigt werden, wenn die für den jeweiligen biologischen, biochemischen, chemischen und/oder physikalischen Prozess wichtigen Bedingungen über- bzw. unterschritten werden, z.B. bei einem zu geringen oder zu hohem Sauerstoffgehalt der Materie, die sich im Reaktor befindet.

Zudem wird eine technische Steuerung der jeweiligen Einheiten ermöglicht, indem eine Einstellung von für den jeweiligen Prozess wesentlichen Parametern während der jeweiligen Prozessabläufe in Echtzeit ermöglicht wird.

Ein weiterer Vorteil liegt darin, dass über die zeitliche Korrelation relevante technische Zusammenhänge der Prozesse unabhängig von einer absoluten Zeit der erfassten Messdaten über die Ausgabeeinheit zugänglich gemacht werden können. Darüber hinaus kann die zeitliche Korrelation über die Dauer der verschiedenen Prozesse erhalten bleiben und über die Anzeigeeinheit angezeigt werden.

Somit wird es einem Benutzer ermöglicht, die technischen Aufgaben der Überwachung von technischen Parametern, die Steuerung der technischen Prozessparameter, die Erfassung technischer Zusammenhänge der verschiedene Prozesse sowie die Überwachung der Auswirkungen verschiedener technischer Parameter auf einen Prozessablauf effizienter und schneller zu bewältigen.

Vorzugsweise umfasst das Computersystem ferner eine Erfassungseinheit, welche eingerichtet ist,
die zeitliche Korrelation zu erkennen, indem sie einen Verlauf der Messdaten der jeweiligen Einheiten miteinander vergleicht; und
eine Anzeige von einander bzw. sich entsprechender Messdaten der jeweiligen Einheiten unabhängig von einer absoluten Zeit ihrer Erfassung über die Anzeigeeinheit zu ermöglichen.

Um die zeitliche Korrelation zu erkennen, kann die Erfassungseinheit zunächst die zwei Einheiten zu einer Gruppe zusammenfassen (Unit Grouping). Dabei ist es unerheblich, ob die Einheiten vom gleichen Typ sind bzw. dieselben Sensoren bzw. Einzelsteuereinheiten umfassen. Somit ermöglicht das System ein freies Unit Grouping, von welchem die Erfassungseinheit die zeitliche Korrelation erkennen kann.

Eine zeitliche Korrelation der Messdaten kann auf eine beliebige Art und Weise, stark in Abhängigkeit der jeweiligen Anwendung und deren Aufgabe oder Fragestellung, erkannt werden. Beispielsweise können Messdaten unterschiedlicher Einheiten
- einen oder mehrere spezifische, d.h. vorbestimmte bzw. vorbestimmbare, absolute Werte bzw. Schwellenwerte und/oder einen oder mehrere relative Werte, z.B. einen pH-Wert, einen CO₂-Wert oder einen O₂-Wert, über- oder unterschreiten;
- einem gemessenen Höchststand bzw. Tiefststand entsprechen;
- einem spezifischen, d.h. vorbestimmten bzw. vorbestimmbaren, Kurvenanstieg, welcher z.B. eine Wachstumsrate darstellen kann, entsprechen;
- einem spezifischen, d.h. vorbestimmten bzw. vorbestimmbaren, Kurvenabstieg, welcher z.B. eine Sterberate darstellen kann, entsprechen;
- nach Durchführung einer deskriptiven univariaten Datenanalyse (z.B. eine Prüfung einer Verteilung, eine Berechnung von Verteilungskenngrößen, eine grafischen Datenanalyse oder eine Korrelationsanalyse) einer spezifischen, d.h. vorbestimmten bzw. vorbestimmbaren, Korrelation zwischen einzelnen Variablen der Messdaten entsprechen;
- nach Durchführung einer Hauptkomponentenanalyse bzw. Principal Component Analysis (PCA) eine spezifische, d.h. vorbestimmte bzw. vorbestimmbare, Datendichte aufweisen;
- nach Durchführung einer Partial Least Squares Regression (PLS) und/oder einer Diskriminanzanalyse und/oder Clusteranalyse spezifische, d.h. vorbestimmte bzw. vorbestimmbare, Strukturen oder Klassen innerhalb der Daten aufweisen; und/oder
- nach Durchführung eines Verfahrens der erweiterten multivariaten Datenanalyse (z.B. Multiway Principal Component Analysis) spezifische, d.h. vorbestimmte bzw. vorbestimmbare, Strukturen in den Daten aufweisen.

Mit anderen Worten sind Merkmale im zeitlichen Verlauf einer Messgröße, mit deren Hilfe die Messdaten zweier oder mehrere Einheiten zueinander zeitlich korreliert werden können (im Weiteren als "Korrelationsmerkmale" bezeichnet), von der jeweiligen Anwendung und deren Aufgabe bzw. Fragestellung abhängig. Solche Korrelationsmerkmale können ein oder mehrere der folgenden sein: das Erreichen, Über- oder Unterschreiten eines gewählten Grenzwerts, ein plötzlicher Anstieg oder plötzlicher Abfall der Messdaten, ein kurzzeitiger und vorübergehender Anstieg oder Abfall (Peak) der Messdaten oder das Erreichen eines vorbestimmten Steigungswertes der Messdaten sein. Beispiele sind das Unter- oder Überschreiten bestimmter Temperaturen oder pH-Werte des Kulturmediums, das Erreichen einer gewünschten Zelldichte, das Erreichen einer gewünschten Wachstumsrate, das Unter- oder Überschreiten von Grenzwerten des Sauerstoff- und/oder Kohlendioxidgehalts des Kulturmediums oder das Erreichen einer gewünschten Konzentration hergestellter Stoffwechselprodukte im Kulturmedium.

Die zeitliche Korrelation der Messdaten hat den Vorteil, dass Messdaten der jeweiligen Einheiten, welche insbesondere nach einem automatischen Vergleich eine spezifische, d.h. vorbestimmte bzw. vorbestimmbare, Bedingung erfüllen, unabhängig von einer absoluten Zeit ihrer Erfassung automatisch über die Anzeigeeinheit dargestellt werden können, so dass Aufschlüsse über einen Zusammenhang dieser Daten ermöglicht werden.

Vorzugsweise kann die zeitliche Korrelation dargestellt werden, indem ein Zeitfenster spezifischer, d.h. vorbestimmter bzw. vorbestimmbarer, Größe derart über die Messdaten der jeweiligen Einheiten angewandt wird, dass Messdaten, die sich im Zeitfenster befinden, unabhängig von einer absoluten Zeit ihrer Erfassung zeitlich korreliert dargestellt werden.

Insbesondere kann das Zeitfenster auf die Messdaten angewandt werden, indem die Anzeigeeinheit einen Schiebebalken (Zoombar) aufweist, an dem ein Schieberegler angebracht ist. Mithilfe einer geeigneten Eingabevorrichtung, z.B. einer Computertastatur, einer Computer-Maus, einer Touch-Screen-Eingabe etc., kann ein Benutzer durch Verschieben des Schiebereglers über den Schiebebalken ein Zeitfenster der Größe N über die Messdaten des Batches schieben. Das so angewandte Zeitfenster wird im Folgenden auch Thumb genannt. Das Zeitfenster der Größe N wird zusätzlich zu der Schiebebalkendarstellung im Anzeigebereich detailliert dargestellt.In der Folge können die jeweiligen Messdaten, die in der Zoombar gezoomt wurden, in Form eines Zeitfensters (Trends) angezeigt werden.

Beispielsweise können die von dem zumindest einen Sensor erfassten Messdaten als Sammlung von Daten bzw. Datensätzen zusammengefasst werden. Beispielsweise kann jeder Batch alle Messdaten beinhalten, die von einer Befüllung einer Einheit, z.B. eines Bioreaktors oder einer Filtrationsanlage, bis zu einer kompletten Entleerung dieser Einheit nach einer spezifischen, d.h. vorbestimmten bzw. vorbestimmbaren, Zeitspanne, z.B. einer Reaktionszeit, einer Wachstumszeit oder nach einem Filterungsprozess, erfasst werden. Die Daten können zeitlich in der Reihenfolge, in der sie hintereinander zusammengefasst werden, angezeigt werden. Optional können den Messdaten zu einem späteren Zeitpunkt Daten zeitlich entsprechend hinzugefügt werden, welche erst nach Beendigung des jeweiligen Prozesses aus einer Auswertung des Stoffes, z.B. nach einer Zentrifugierung des Stoffes, ermittelt werden können (Offline-Daten). Insbesondere können die Messdaten, die im Batch gespeichert werden, im Folgenden auch Batchdaten genannt, zur Zertifizierung des jeweiligen Prozesses verwendet werden und so entsprechenden Standards zur Prozessherstellung genügen.

Da es ab einer bestimmten Dichte von Daten nicht möglich ist, alle Daten, die in einem Batch zusammengefasst sind, gleichzeitig anzuzeigen, kann über das Computersystem eine Voreinstellung eingestellt werden, nach welcher die Anzeigeeinheit immer die letzten N Daten bzw. Datensätze, die erfasst und zum Batch hinzugefügt wurden, anzeigt, wobei N eine spezifische, d.h. vorbestimmte bzw. vorbestimmbare, Anzahl von Daten bzw. Datensätzen definiert. Dies wird mit einstellbaren Zeitspannen gelöst. Das Anzeigen aller Messdaten, unabhängig von der eingestellten Zeitspanne, kann dadurch ermöglicht werden, indem ein MinMax Balken für diesen Zeitraum hinterlegt ist.

Die zeitliche Korrelation kann dargestellt werden, indem ein Zeitfenster spezifischer, d.h. vorbestimmter bzw. vorbestimmbarer, Größe derart auf die Messdaten der jeweiligen Einheiten angewandt wird, dass Messdaten, die sich im Zeitfenster befinden, unabhängig von einer absoluten Zeit ihrer Erfassung zeitlich korreliert dargestellt werden. Diese zeitliche Korrelation bzw. Synchronisation der Messdaten kann dabei in der Anzeigeeinheit erhalten bleiben, auch wenn neue Messdaten im Batch aufgenommen werden. Mit anderen Worten kann die Anzeigeeinheit lediglich die zeitlich korrelierten Daten, die sich innerhalb des Zeitfensters befinden, während zweier aktuell laufender Prozesse beibehalten und anzeigen. Beispielsweise können Messdaten, die von den Sensoren der zwei Einheiten erfasst wurden und welche nach einem automatischen Vergleich der Messdaten eine spezifische, d.h. vorbestimmte bzw. vorbestimmbare, Bedingung erfüllen, unabhängig von der absoluten Zeit ihrer Erfassung über das Zeitfenster während des weiteren Verlaufs der Prozesse zeitlich korreliert über die Anzeigeeinheit angezeigt werden. In diesem Fall werden die neuen Daten bzw. Datensätze, die von den Sensoren erfasst und zum entsprechenden Batch hinzugefügt werden, nicht durch die Anzeigeeinheit dargestellt.

In einem anderen Beispiel kann die zeitliche Korrelation eingestellt werden, indem Messdaten, die von Sensoren der jeweiligen Einheiten in unterschiedlichen zeitlichen Abständen gemessen wurden, in zeitliche Korrelation miteinander gebracht werden. Insbesondere kann die Größe des Zeitfensters, welche auf den jeweiligen Batch angepasst wird, derart angepasst werden, dass sich die Messdaten in ihren zeitlichen Abständen entsprechen.

Vorzugsweise kann die zeitliche Korrelation in Abhängigkeit einer Eingabe eines Benutzers eingestellt werden.

Beispielsweise kann ein Benutzer über die Zoombar, an welcher ein Schieberegler angebracht ist, mithilfe einer geeigneten Eingabevorrichtung, z.B. einer Computertastatur, einer Computer-Maus, einer Touch-Screen-Eingabe etc., einen Bereich der über die Anzeigeeinheit angezeigten Messdaten durch Verschieben des Schiebereglers auswählen.

Dies hat den Vorteil, dass eine verbesserte, kontinuierliche Benutzer-Maschinen-Interaktion erzielt wird, da der Benutzer die Anzeige der Messdaten der jeweiligen Einheiten über einen zeitlichen Verlauf mithilfe der Anzeigeeinheit steuern und zeitlich korreliert anzeigen kann.

Zudem wird es dem Benutzer ermöglicht, eine Breite des Thumbs und somit eine Anzahl an anzuzeigenden Messdaten einzustellen, indem er mithilfe einer geeigneten Eingabevorrichtung, z.B. einer Computertastatur, einer Computer-Maus, einer Touch-Screen-Eingabe etc., einen Punkt der Zoombar, der sich außerhalb des Thumbs befindet, auswählt. So kann jenes Ende des Thumbs, welches sich auf der Seite des ausgewählten Punktes befindet, zum Punkt hin erweitert werden.

Insbesondere kann ein Benutzer mithilfe einer geeigneten Eingabevorrichtung die Breite des Thumbs vergrößern, verkleinern sowie den Thumb innerhalb der Zoombar beliebig nach rechts und links bewegen. Dies hat den Vorteil, dass die Anzeige der technischen Zustände der einzelnen Einheiten sowie die Anzeige relevanter technischer Zusammenhänge unabhängig von der absoluten Zeit der Erfassung der Messwerte gesteuert werden kann, während die Prozesse in den Einheiten laufen.

Vorzugsweise werden die Messdaten der zwei Einheiten über die Anzeigeeinheit in jeweils einem Anzeigebereich angezeigt, wobei die Einstellung zudem beinhalten kann, dass der Benutzer in jedem Anzeigebereich ein Zeitfenster jeweils an einen spezifischen, d.h. vorbestimmten bzw. vorbestimmbaren, Zeitpunkt der Messdaten mithilfe eines Schiebereglers, welcher das Zeitfenster bzw. Thumb für die anzuzeigenden Messwerte einstellt und welcher entlang der Messdaten im jeweiligen Anzeigebereich verschoben werden kann, anwendet.

Beispielsweise kann sich ein Benutzer manuell mithilfe des Schiebereglers Messdaten, die über eine Temperatur während des Prozesses einer Züchtung von Hefezellen in Reinkultur Auskunft geben, anzeigen lassen, wobei der Prozess noch immer läuft und somit immer neue Messdaten im Batch gespeichert werden. Findet der Benutzer einen für ihn relevanten Temperaturwert, kann sich der Benutzer die Messkurve von diesem relevanten Temperaturwert an anzeigen lassen, um den weiteren Temperaturverlauf von relevanten Zeitpunkt an zu verfolgen und zwar unabhängig vom absoluten Zeitpunkt der Erfassung der Messwerte.

Beispielsweise können die Messdaten jeder Einheit in einem eigenen Batch zusammengefasst werden. Entsprechend können die Messdaten jeder Einheit in jeweils einem spezifischen, d.h. vorbestimmten bzw. vorbestimmbaren, Bereich der Anzeigeeinheit, im Folgenden auch Anzeigebereich genannt, angezeigt werden. In diesem Beispiel kann jeweils eine Zoombar pro Anzeigeeinheit bereitgestellt werden.

Beispielsweise kann jedem Anzeigebereich eine Zoombar entsprechen. Somit kann ein Benutzer durch Verschieben eines jeweiligen Schiebereglers in jedem Anzeigebereich eigene Messdaten zur Anzeige auswählen.

Dies hat den Vorteil, dass ein Benutzer Messdaten über einen spezifischen, d.h. vorbestimmten bzw. vorbestimmbaren, zeitlichen Verlauf jeder der beiden Einheiten individuell und unabhängig voneinander steuern und/oder über die Anzeigeeinheit anzeigen lassen kann. Somit können dem Benutzer relevante technische Zusammenhänge der unterschiedlichen Prozesse unabhängig von einer absoluten Zeit von jeweils erfassten Messdaten über die Anzeigeeinheit zugänglich gemacht werden. Zudem kann mithilfe der Zeitfenster bzw. Thumbs die zeitliche Korrelation der technischen Zusammenhänge über eine Dauer der verschiedenen Prozesse erhalten bleiben.

Vorzugsweise umfasst das Computersystem zusätzlich ein Steuerungsmodul, welches eingerichtet ist, zumindest zwei Einheiten zu einer Gruppe zusammenzufassen. Die Gruppe kann über das Steuerungsmodul gesteuert werden. Darüber hinaus lässt sich über das Steuerungsmodul eine Regelung der Messdaten durchführen.

Beispielsweise kann das Steuerungsmodul die Zeitfenster derart miteinander verlinken bzw. in Verhältnis zueinander stellen bzw. verbinden, dass zeitlich korrelierte Messdaten der jeweiligen Einheiten unabhängig von einer absoluten Zeit ihrer Erfassung automatisch über die Anzeigeeinheit dargestellt werden können. Dazu kann die Zoombar automatisch an die Bereiche der jeweiligen Batches bewegt werden, an denen die Messdaten die Bedingung erfüllen.

Der Messdatenverlauf eines Prozesses wird im Folgenden auch Trend genannt. Bei einer Synchronisation der Trends kann eine Breite eines Trends über eine erste Zoombar, welche N Messdaten eines Batches darstellt, an eine Breite eines zweiten Trends über eine zweite Zoombar, welche mehr bzw. weniger als N Messdaten eines Batches darstellt, automatisch angepasst werden, so dass innerhalb der Trends dieselbe Zeitspanne, d.h. dieselbe Anzahl an Messdaten, angezeigt wird.

Dies hat den Vorteil, dass eine synchrone Anzeige unterschiedlicher, wichtiger Prozessfaktoren bzw. eine Änderung ein oder mehrerer solcher Prozessfaktoren in Echtzeit auf eine einfache und effiziente Weise gesteuert werden kann.

Vorzugsweise ist das Steuerungsmodul zudem eingerichtet, zeitlich korrelierte Zeitfenster der jeweiligen Einheiten derart miteinander zu verlinken, dass ein Benutzer die mehreren Zeitfenster mithilfe eines einzigen Schiebereglers steuern kann, indem er jeweils nur ein Zeitfenster mithilfe des Schiebereglers über die Zoombar verschiebt. Beispielsweise kann jede Aktion, welche automatisch oder von einem Benutzer mittels ersten Zoombar (Aktor) erzeugt wird, in der zweiten Zoombar (Reaktor) reflektiert werden. Für den Fall, dass die durchgeführte Aktion beim Reaktor nicht komplett, sondern nur teilweise angewandt werden kann, kann die Aktion im Reaktor entsprechend teilweise durchgeführt werden. Für den Fall, dass die Aktion im Reaktor nicht durchgeführt werden kann, kann die Aktion im Aktor rückgängig gemacht werden.

Eine Aktion kann beispielsweise nur teilweise durchgeführt werden, wenn eine Verbreiterung eines Thumbs in einem Aktor nach links initiiert wird, die prinzipiell am Reaktor auch möglich ist, allerdings nicht in dem initiierten Ausmaß. In diesem Fall wird die initiierte Aktion der Verbreiterung des Thumbs nur bis zu dem Bereich durchgeführt, welcher für den Reaktor (und somit per Definition für den Aktor) möglich ist. Ist im Reaktor eine Verbreiterung des Thumbs unmöglich, wird die Aktion nicht durchgeführt.

Das Verlinken der Zeitfenster hat den technischen Vorteil einer verbesserten Steuerung der jeweiligen Prozessparameter. Zudem werden dem Benutzer relevante technische Zusammenhänge unterschiedlicher Prozesse unabhängig von einer absoluten Erfassung entsprechender Messwerde zugänglich gemacht, was zu dem technischen Vorteil einer verbesserten Analyse der jeweiligen technischen Prozessparameter führt.

Vorzugsweise ist das Steuerungsmodul zudem eingerichtet, Prozesse, die an dem Stoff der jeweiligen Einheiten durchgeführt werden sollen, zum selben Zeitpunkt, unabhängig vom absoluten Zeitpunkt, zu starten.

Dies kann entweder automatisch erfolgen, indem ein Zeitpunkt für den Start vorbestimmt wird, oder manuell durch eine geeignete Benutzereingabe.

Vorzugsweise handelt es sich bei dem Stoff um eine Zusammensetzung aus flüssigen und gasförmigen Fluide, z.B. einer begasten wässrigen Lösung aus Nährstoffen, dem die zu kultivierenden Mikroorganismen zugefügt werden. Zusätzlich kann die Zusammensetzung, z.B. zur Kultivierung adhärenter Zellen, Festkörper enthalten, auf denen sich die Zellen ansiedeln können. Der Stoff kann jedoch auch rein chemischer Natur sein und keine Mikroorganismen enthalten.

Gemäß eines weiteren Aspekts der vorliegenden Erfindung wird die zugrunde liegende Aufgabe durch ein computer-implementiertes Verfahren gemäß Anspruch 8 zur Steuerung, Erfassung, Regelung und/oder Analyse von biologischen, biochemischen, chemischen und/oder physikalischen Prozessen gelöst, wobei das Verfahren folgende Funktionen beinhaltet:
Bereitstellen von zumindest zwei Einheiten bzw. Units, welche eingerichtet sind, einen Stoff bzw. eine Materie aufzunehmen, um an diesem Stoff zumindest einen biologischen, biochemischen, chemischen und/oder physikalischen Prozess durchzuführen, wobei jede der Einheiten zumindest einen Sensor aufweist;
Erfassen, durch die Sensoren, von Messdaten betreffend den jeweiligen Prozess; und Anzeigen, durch eine Anzeigeeinheit, der Messdaten der zwei Einheiten in einer zeitlichen Korrelation;
wobei die zeitliche Korrelation Aufschlüsse über einen Zusammenhang der dargestellten Messdaten ermöglicht.

Somit wird es einem Benutzer ermöglicht, die technischen Aufgaben der Überwachung der technischen Prozessparameter, der Steuerung der technischen Prozessparameter, der Erfassung technischer Zusammenhänge der jeweiligen Prozesse sowie der Überwachung von Auswirkungen verschiedener technischer Prozessparameter auf einen Prozessablauf effizienter und schneller zu bewältigen.

Vorzugsweise beinhaltet das Verfahren zudem folgende Funktionen:
Erkennen, durch eine Erfassungseinheit, der zeitlichen Korrelation, indem ein Verlauf der Messdaten der jeweiligen Einheiten miteinander verglichen wird; und
Anzeigen, durch die Anzeigeeinheit, von entsprechenden bzw. sich entsprechenden Messdaten der jeweiligen Einheiten unabhängig von einer absoluten Zeit ihrer Erfassung.

Vorzugsweise kann die zeitliche Korrelation dargestellt werden, indem ein Zeitfenster spezifischer, d.h. vorbestimmter bzw. vorbestimmbarer, Größe derart über die Messdaten der jeweiligen Einheiten angewandt wird, dass Messdaten, die sich im Zeitfenster befinden, unabhängig von einer absoluten Zeit ihrer Erfassung zeitlich korreliert dargestellt werden.

Vorzugsweise kann die zeitliche Korrelation in Abhängigkeit einer Eingabe eines Benutzers eingestellt werden.

Vorzugsweise können die Messdaten der zwei Einheiten über die Anzeigeeinheit in jeweils einem Anzeigebereich angezeigt werden.

Vorzugsweise kann die Einstellung zudem beinhalten, dass der Benutzer in jedem Anzeigebereich ein Zeitfenster jeweils an einen spezifischen, d.h. vorbestimmten bzw. vorbestimmbaren, Zeitpunkt der Messdaten mithilfe eines Schiebereglers, welcher entlang der Messdaten im jeweiligen Anzeigebereich verschoben werden kann, anwendet.

Vorzugsweise beinhaltet das Verfahren zusätzlich folgende Funktion:
Zusammenfassen, über ein Steuerungsmodul, von zumindest zwei Einheiten zu einer Gruppe, so dass die Gruppe über das Steuerungsmodul gesteuert werden kann. Darüber hinaus lässt sich über das Steuerungsmodul eine Regelung der Messdaten durchführen.

Vorzugsweise beinhaltet das Verfahren zusätzlich folgende Funktion:
Verlinken von Zeitfenster, über das Steuerungsmodul, die über Messdaten der jeweiligen Einheiten angewandt sind und jeweils in einem Anzeigebereich angezeigt werden, so dass ein Benutzer die Zeitfenster mithilfe eines Schiebereglers steuern kann, indem er die Zeitfenster synchron über die Messdaten der jeweiligen Einheiten verschiebt.

Vorzugsweise beinhaltet das Verfahren zusätzlich folgende Funktion:
Starten der Prozesse, über das Steuerungsmodul, die an dem Stoff der jeweiligen Einheiten durchgeführt werden sollen, zum selben Zeitpunkt, unabhängig vom absoluten Zeitpunkt.

Gemäß eines weiteren Aspekts der vorliegenden Erfindung wird die zugrunde liegende Aufgabe durch ein Computerprogrammprodukt gemäß Anspruch 13 gelöst, welches Programmteile umfasst, welche, wenn in einem Computer geladen, zur Durchführung eines computer-implementierten Verfahrens geeignet sind, wobei das computer-implementierte Verfahren folgende Funktionen umfasst:
Erkennen, durch eine Erfassungseinheit, einer zeitlichen Korrelation, indem ein Verlauf der Messdaten der jeweiligen Einheiten miteinander verglichen wird; und
Anzeigen, durch die Anzeigeeinheit, von Messdaten, die sich entsprechen, in zeitlich entsprechender Weise, unabhängig von einer absoluten Zeit ihrer Erfassung.

Vorzugsweise kann die zeitliche Korrelation dargestellt werden, indem ein Zeitfenster spezifischer, d.h. vorbestimmte bzw. vorbestimmbarer, Größe derart über die Messdaten der jeweiligen Einheiten angewandt wird, dass Messdaten, die sich im Zeitfenster befinden, unabhängig von einer absoluten Zeit ihrer Erfassung zeitlich korreliert dargestellt werden.

Vorzugsweise kann die zeitliche Korrelation von einem Benutzer manuell eingestellt werden.

Vorzugsweise können die Messdaten der zwei Einheiten über die Anzeigeeinheit in jeweils einem Anzeigebereich angezeigt werden.

Vorzugsweise kann die manuelle Einstellung zudem beinhalten, dass der Benutzer in jedem Anzeigebereich ein Zeitfenster jeweils an einen spezifischen, d.h. vorbestimmten bzw. vorbestimmbaren, Zeitpunkt der Messdaten mithilfe eines Schiebereglers, welcher entlang der Messdaten im jeweiligen Anzeigebereich verschoben werden kann, anwendet.

Vorzugsweise beinhaltet das Verfahren zusätzlich folgende Funktion:
Zusammenfassen, über ein Steuerungsmodul, von zumindest zwei Einheiten zu einer Gruppe, so dass die Gruppe gleichzeitig über das Steuerungsmodul gesteuert wird.

Vorzugsweise beinhaltet das Verfahren zusätzlich folgende Funktion:
Verlinken von Zeitfenstern, über das Steuerungsmodul, die über Messdaten der jeweiligen Einheiten angewandt sind und jeweils in einem Anzeigebereich angezeigt werden, so dass ein Benutzer die Zeitfenster mithilfe eines Schiebereglers steuern kann, indem er die Zeitfenster synchron über die Messdaten der jeweiligen Einheiten verschiebt.

Vorzugsweise beinhaltet das Verfahren zusätzlich folgende Funktion:
Starten der Prozesse, über das Steuerungsmodul, die an dem Stoff der jeweiligen Einheiten durchgeführt werden sollen, zum selben Zeitpunkt.

Gemäß eines weiteren Aspekts der vorliegenden Erfindung wird die zugrundeliegende Aufgabe durch ein Graphical User Interface (GUI) bzw. eine grafische Benutzerschnittstelle zur Steuerung, Erfassung, Regelung und/oder Analyse von biologischen, biochemischen, chemischen und/oder physikalischen Prozessen gemäß Anspruch 14 gelöst, wobei die GUI umfasst:
zumindest zwei Anzeigebereiche spezifischer, d.h. vorbestimmter bzw. vorbestimmbarer, Größe und spezifischer, d.h. vorbestimmter bzw. vorbestimmbarer, Position, wobei jeder Anzeigebereich einer Einheit bzw. Unit zugeordnet ist, welche eingerichtet ist, einen Stoff bzw. eine Materie aufzunehmen, um an diesem Stoff zumindest einen biologischen, biochemischen, chemischen und/oder physikalischen Prozess durchzuführen,
wobei jede der Einheiten zumindest einen Sensor aufweist, welcher eingerichtet ist, Messdaten über den Prozess zu erfassen,
wobei in jedem Anzeigebereich die Messdaten der zwei Einheiten jeweils in einer zeitlichen Korrelation dargestellt werden, welche Aufschlüsse über einen Zusammenhang der dargestellten Messdaten ermöglichen.

Bevorzugte Ausführungsformen werden im Folgenden mit Bezug auf begleitende Zeichnungen beispielhaft beschrieben. Es wird angemerkt, dass selbst wenn Ausführungsformen separat beschrieben sind, einzelne Merkmale davon zu zusätzlichen Ausführungsformen kombiniert werden können. Es zeigen:
- **Figur 1**: ein beispielhaftes Computersystem zur Steuerung, Erfassung, Regelung und/oder Analyse von biologischen, biochemischen, chemischen und/oder physikalischen Prozessen;
- **Figur 2**: einen beispielhaften Aufbau einer Einheit;
- **Figur 3**: einen Überblick über eine beispielhafte Struktur und Zusammenhänge von Messdaten einer Einheit, die zu einem Batch zusammengefasst werden;
- **Figur 4**: eine schematische Darstellung zweier Einheiten, die zu einer Gruppe zusammengefasst werden;
- **Figur 5**: eine beispielhafte Anzeige über eine von Messdaten, welche über die Anzeigeeinheit ausgegeben werden;
- **Figur 6**: eine beispielhafte Anordnung zweier Thumbs;
- **Figur 7**: eine beispielhaft Anzeige von Messdaten vor einer Synchronisation;
- **Figur 8**: die Anzeige von Messdaten wie in Figur 7 nach der Synchronisation;
- **Figur 9**: ein Prozessablaufdiagramm zur Synchronisation;
- **Figur 10**: ein Prozessflussdiagramm, das den Prozess des Verlinkens der Zoombars, wie bezüglich der Figuren 7 und 8 beschrieben, visualisiert;
- **Figur 11**: ein beispielhaftes System zur Implementierung der Erfindung.

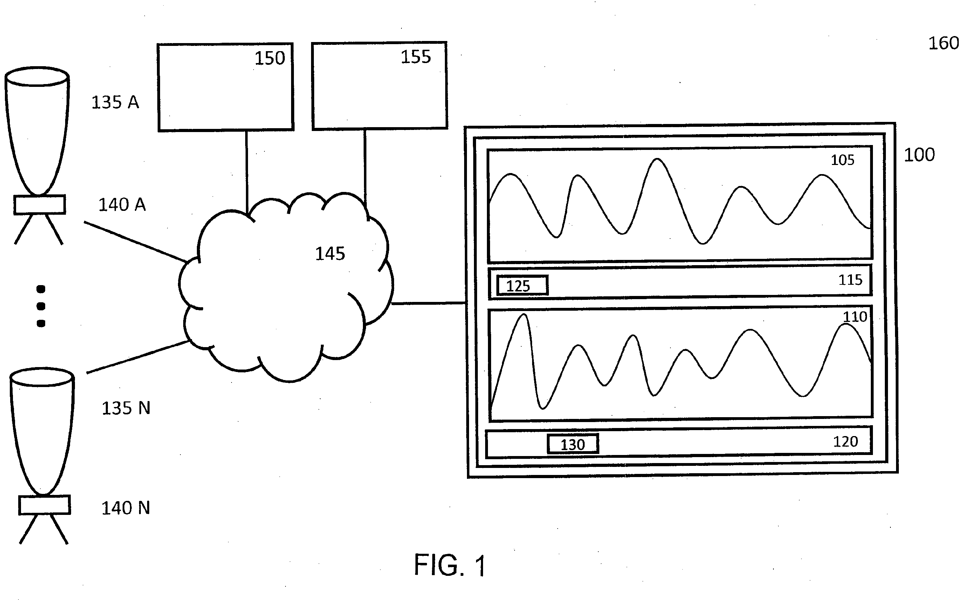

**Figur 1** zeigt Computersystem 160 zur Steuerung, Erfassung, Regelung und/oder Analyse von biologischen, biochemischen, chemischen und/oder physikalischen Prozessen. Das in Figur 1 gezeigte Computersystem 160 umfasst mehrere, zumindest jedoch zwei Einheiten 135 A bis 135 N (135 A-N), die eingerichtet sind, einen Stoff bzw. eine Materie aufzunehmen, um an diesem Stoff zumindest einen biologischen, biochemischen, chemischen und/oder physikalischen Prozess durchzuführen. Jede der Einheiten 135 A-N weist zumindest einen Sensor 140 A bis 140N (140 A-N) auf, der eingerichtet ist, Messdaten betreffend den Prozess zu erfassen. Zudem umfasst das Computersystem 160 zumindest eine Anzeigeeinheit 100, über welche die Messdaten der Einheiten 135 A-N jeweils in einer zeitlichen Korrelation dargestellt werden, welches Aufschlüsse über einen Zusammenhang der dargestellten Messdaten ermöglicht. Beispielsweise können die Messdaten der jeweiligen Einheiten 135 A-N in eigenen Anzeigebereichen 105, 110 angezeigt werden. Zudem kann der Benutzer über die Anzeigeeinheit 100 mithilfe eines jeweiligen Schiebereglers 125, 130 ein Zeitfenster über die entsprechenden Zoombars 113,120 an die Messdaten der jeweiligen Batches anwenden, so dass der Verlauf der Messdaten ausgehend vom jeweiligen Zeitfenster unabhängig von einer absoluten Zeit der Messdaten über die Anzeigeeinheit 100 ausgegeben werden.

Insbesondere kann das Computersystem 160 zur Steuerung, Erfassung, Regelung bzw. Steuerung und/oder Analyse von biologischen, biochemischen, chemischen und/oder physikalischen Prozessen als Supervisory Control and Data Acquisition (SCADA)- System ausgelegt sein. In der Folge kann ein technischer Prozess computermäßig überwacht, gesteuert und/oder geregelt werden.

Bei SCADA-Systemen können insbesondere Automationen entsprechend dem Open System Interconnection (OSI)-Schichtenmodell in mehrere Schichten unterteilt werden, wobei das OSI-Schichtenmodell insbesondere ein Referenzmodell für herstellerunabhängige Kommunikationssysteme bzw. eine Design-Grundlage für Kommunikationsprotokolle und Computernetze darstellt, welches einem ISO Standard entspricht. Das OSI-Schichtenmodell besteht aus 7 Schichten:
7. Schicht: Anwendung: Funktionen für Anwendungen, sowie die Dateneingabe und - ausgabe.
6. Schicht: Darstellung: Umwandlung der systemabhängigen Daten in ein unabhängiges Format.
5. Schicht: Kommunikation: Steuerung der Verbindungen und des Datenaustauschs.
4. Schicht: Transport: Zuordnung der Datenpakete zu einer Anwendung.
3. Schicht: Vermittlung: Routing der Datenpakete zum nächsten Knoten.
2. Schicht: Sicherung: Segmentierung der Pakete in Frames und Hinzufügen von Prüfsummen.
1. Schicht: Bitübertragung: Umwandlung der Bits in ein zum Medium passendes Signal und physikalische Übertragung.

Jede Schicht hat innerhalb der Kommunikation zwischen zwei Systemen eine bestimmte Aufgabe zu erfüllen. Für jede Schicht werden Funktionen und Protokolle definiert, die bestimmte Aufgaben bei der Kommunikation zwischen zwei Systemen erfüllen müssen. Bei der Kommunikation zwischen zwei Systemen durchläuft die Kommunikation oder der Datenfluss insbesondere alle 7 Schichten des OSI-Schichtenmodells zweimal. Einmal beim Sender und einmal beim Empfänger. Je nachdem, wie viele Zwischenstationen die Kommunikationsstrecke aufweist, durchläuft die Kommunikation auch mehrmals das Schichtenmodell. Protokolle sind in diesem Zusammenhang eine Sammlung von Regeln zur Kommunikation auf einer bestimmten Schicht des OSI-Modells. Die Endgeräte der Endsysteme und das Übertragungsmedium sind aus dem OSI-Modell ausgeklammert. Allerdings können die Endgeräte in der Anwendungsschicht und das Übertragungsmedium in der Bitübertragungsschicht vorgegeben sein. Die Protokolle einer Schicht sind insbesondere zu den Protokollen der über- und untergeordneten Schichten weitestgehend transparent, so dass die Verhaltensweise eines Protokolls sich wie bei einer direkten Kommunikation mit dem Gegenstück auf der Gegenseite darstellt. Die Übergänge zwischen den Schichten können insbesondere Schnittstellen sein, die von den Protokollen verstanden werden müssen. Weil manche Protokolle für ganz bestimmte Anwendungen entwickelt wurden, kommt es auch vor, dass sich Protokolle über mehrere Schichten erstrecken und mehrere Aufgaben abdecken. Es kann dann sogar sein, dass in manchen Verbindungen einzelne Aufgaben mehrfach ausgeführt werden.

Der Begriff SCADA bezieht sich gewöhnlich auf zentrale/dezentrale Systeme, die gesamte Installationen überwachen, visualisieren und/oder steuern bzw. regeln. Der größte Teil der Regelung wird automatisch durch Fernbedienungsterminals (Remote Terminal Unit, RTU) oder durch Speicherprogrammierbare Steuerungen (SPS, auch Programmable Logic Controller, PLC genannt) beziehungsweise Level-1-Automationen durchgeführt. Die Aufgabe der Level-2-Automation ist es, die Funktion der Level-1-Automation zu optimieren sowie Stellgrößen und/oder Sollwerte auszugeben. Die Level-3-Automation dient hingegen der Planung, Qualitätssicherung und/oder Dokumentation.

Die Kommunikation innerhalb eines SCADA-Systems kann auf der Basis von TCPbasierten Internettechniken erfolgen, wobei auch eine oder mehrere serielle Verbindungen in Form von Punkt-zu-Punkt-Kommunikationen und/oder Feldbussystemen möglich sind.

Die Datenerfassung beginnt gewöhnlich mit dem Level 1 und enthält die Koppelung an Messgeräte und Statusinformationen wie Schalterstellungen, die von dem SCADA-System erfasst werden. Die Daten werden dann in einer benutzerfreundlichen Darstellung präsentiert und ermöglichen es, steuernd in den Prozess einzugreifen.

SCADA-Systeme implementieren typischerweise eine verteilte Datenbasis, die Datenpunkte beinhaltet. Ein Datenpunkt enthält einen Ein- oder Ausgangswert, der durch das System überwacht und/oder gesteuert wird. Datenpunkte können physikalisch berechnet werden. Ein physikalischer Datenpunkt stellt einen Eingang oder Ausgang dar, während ein berechneter Punkt durch mathematische Operationen aus dem Zustand des Systems hervorgeht. Normalerweise werden Datenpunkte als eine Kombination von Werten mit Zeitstempel behandelt. Eine Serie von Datenpunkten ermöglicht die historische Auswertung.

Hierbei kann die zeitliche Korrelation von Datenpunkten aus verschiedenen Einheiten 135 A-N vorteilhaft zur Anwendung kommen, um Zusammenhänge der entsprechenden Datenpunkten bzw. Messdaten zu erfassen. Dies ermöglicht es einem Benutzer vorteilhaft, aufgrund einer Darstellung der Messdaten in einer zeitlich korrelierten Weise entsprechenden Einfluss auf den Prozess bzw. die Prozesse zu nehmen, z.B. durch Einstellung von entsprechenden Prozessparametern, Triggern von spezifischen Abläufen o.ä.

Jede zeitliche Korrelation umfasst ein horizontales Verschieben bzw. Ausrichten der Messdaten der jeweiligen Einheiten 135 A-N, so dass die Messdaten beliebig aufeinander bezogen werden können.

Eine *Einheit* 135 A-N *bzw. Unit bzw. Teilanlage* umfasst insbesondere zumindest einen Reaktor, z.B. zumindest einen Bioreaktor und/oder zumindest einen chemischen Reaktor, zumindest einen Sensor 140 A-N sowie optional ein oder mehrere Einzelsteuereinheiten und Prozessgeräte. Jede Einheit 135 A-N kann in ihrer Gesamtheit aus logischer Sicht einen Teil einer Produktionsstätte, z.B. in einem Produktionswerk zur Herstellung von Hefekulturen, darstellen, in welcher ein oder mehrere Prozessaktivitäten durchgeführt werden können. Jede Einheit 135 A-N generiert aus den Messdaten, die von dem Sensor 140 A-N und optional von Einzelsteuereinheiten zu einem gegeben Zeitpunkt erfasst werden, vorzugsweise nur einen Batch. Jede Einheit 135 A-N kann unabhängig von jeder anderen Einheit 135 A-N agieren. So kann mit jeder Einheit 135 A-N ein spezifischer, d.h. vorbestimmter bzw. vorbestimmbarer, Prozess abgebildet werden, damit alle zu einer Prozessaufzeichnung bzw. Prozessüberwachung benötigten Messdaten mithilfe von zumindest einem Sensor 140 A-N erfasst und gleichzeitig angezeigt werden können. Jede Einheit 135 A-N kann einem spezifischen Prozess entsprechend individuell, je nach erforderlichem Prozess, aus einer beliebigen Kombination der o.g. Komponenten, zusammengestellt werden.

Alternativ oder zusätzlich zum Reaktor kann eine Einheit 135 A-N insbesondere zumindest einen Filter beinhalten, welcher zur Filtration eines Mediums verwendet werden kann. Beispielsweise kann es sich dabei um einen Filter zur Cross-Flow-Filtration bzw. Tangential-Flow-Filtration bzw. Querstromfiltration handeln, bei welcher Flüssigkeiten beispielsweise für die Lebensmittel- und/oder Pharmaindustrie filtriert werden. Dabei wird eine zu filtrierende Suspension mit einer hohen Geschwindigkeit parallel zu einer Membran oder eines Filtermediums gepumpt, wobei ein Feststoff bzw. Permeat quer zur Fließrichtung abgezogen wird. Die Cross-Flow-Filtration kann beispielsweise in der Mikrofiltration, Ultrafiltration, Nanofiltration, Gastrennung, Pervaporation und/oder Umkehrosmose angewandt werden. Jede dieser Einheiten 135 A-N umfasst zumindest einen Sensor 140 A-N sowie optional ein oder mehrere Einzelsteuereinheiten und Prozessgeräte. Jede Einheit 135 A-N kann in ihrer Gesamtheit aus logischer Sicht einen Teil einer Produktionsstätte, z.B. in einem Wasseraufbereitungswerk zur Entfernung von Schwermetallen in der Wasseraufbereitung, darstellen, in welchem ein oder mehrere Prozessaktivitäten durchgeführt werden können. Jede Einheit 135 A-N generiert aus Messdaten, die von dem Sensor 140 A-N und optional den Einzelsteuereinheiten zu einem gegeben Zeitpunkt erfasst werden, vorzugsweise nur einen Batch. Jede Einheit 135 A-N kann unabhängig von jeder anderen Einheit 135 A-N agieren. So kann mit jeder Einheit 135 A-N ein spezifischer Prozess abgebildet werden, damit alle zu einer Prozessaufzeichnung bzw. Prozessüberwachung benötigten Messdaten mithilfe von zumindest einem Sensor 140 A-N erfasst und gleichzeitig angezeigt werden können. Jede Einheit 135 A-N kann einem spezifischen Prozess entsprechend individuell je nach erforderlichem Prozess aus einer beliebigen Kombination der o.g. Komponenten, zusammengestellt werden.

Ein *Sensor* 140 A-N ist insbesondere ein technisches Bauteil, welches bestimmte biologische, biochemische, chemische und/oder physikalische Eigenschaften seiner Umgebung als Messgröße quantitativ erfassen kann. Dabei werden die biologischen, biochemischen, chemischen und/oder physikalischen Eigenschaften in ein elektrisches und/oder optisches Signal umgewandelt. Das elektrische Signal kann dann abgetastet werden. Dabei kann für jede biologische, biochemische, chemische und/oder physikalische Eigenschaft ein numerisches Messdatum bzw. ein numerischer Messwert erzeugt werden. Somit kann der Sensor 140 A-N einen Datenstrom der entsprechenden Messdaten erstellen.

Jede Einheit 135 A-N weist insbesondere zumindest einen Sensor 140 A-N auf, welcher eingerichtet ist, spezifische, d.h. vorbestimmte bzw. vorbestimmbare, Messdaten bezüglich des Prozesses zu erfassen. Beispielsweise kann es sich dabei um einen Gassensor, z.B. einen O₂- bzw. CO₂-Sensor, einen pH-Sensor, einen Glucose-Sensor, einen Lactat-Sensor und/oder um eine beliebige Kombination der Sensoren handeln, der bzw. die eingerichtet sind, eine geeignete Messgröße im Hinblick auf den durchzuführenden Prozess zu erfassen. Optional kann jede Einheit 135 A-N zudem ein oder mehrere Einzelsteuereinheiten aufweisen.

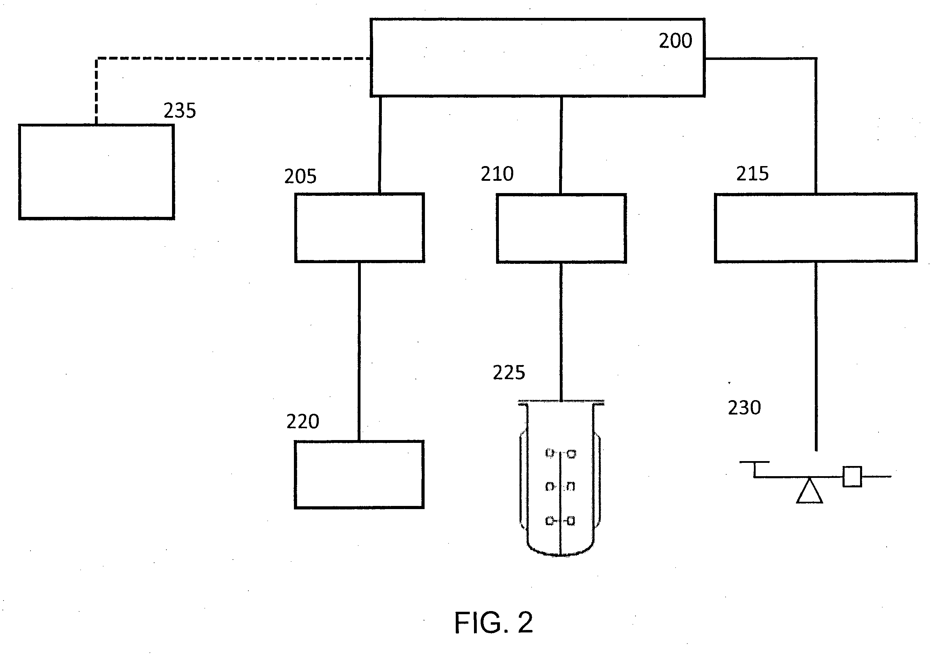

Ein *Bioreaktor* bzw. Fermenter 225 als eine spezielle Ausführungsform einer Einheit 135 A-N (wie weiter unten mit Bezug auf **Figur 2** beschrieben) ist insbesondere ein Behälter, welcher eingerichtet ist, Zellen, Mikroorganismen und/oder kleine Pflanzen zu kultivieren bzw. zu fermentieren. In dem Bioreaktor 225 werden biologische Prozesse, z.B. eine Biokonversion oder eine Biokatalyse, in technischen Einrichtungen genutzt bzw. nutzbar gemacht. Dabei wird versucht, möglichst optimale Kultivierungsbedingungen mithilfe von wichtigen Faktoren, die in dem Bioreaktor 225 kontrollierbar bzw. steuerbar sind, zu erzielen. Kontrollierbare Faktoren können z.B. eine Zusammensetzung einer Nährlösung bzw. eines Substrates, eine Sauerstoffzufuhr, eine Sterilität oder ein pH-Wert sein. Zweck einer Kultivierung in dem Bioreaktor 225 kann eine Gewinnung von Zellen, eine Gewinnung von Zellbestandteilen oder eine Gewinnung von Stoffwechselprodukten bzw. Metaboliten sein, welche daraufhin beispielsweise als Wirkstoff in der pharmazeutischen Industrie oder Grundchemikalie in der chemischen Industrie verwendet werden können. Beispiele für solche Bioreaktoren sind z.B. autoklavierbare Bioreaktoren, in-situ sterilisierbare Bioreaktoren, Einweg-Bioreaktoren, Rührkesselreaktoren, Festbettreaktoren oder Photobioreaktoren. Auch die Herstellung von Alkoholika bzw. alkoholischen Getränken, z.B. Bier, kann in Bioreaktoren, z.B. einem Braukessel, stattfinden. Insbesondere unterliegen Bioreaktoren keinerlei spezifischen Einschränkungen. Sie umfassen beispielsweise Bioreaktoren zur Kultur von Zellen, wie Bakterien-, Hefe-, Insekten, Pflanzen- oder Säugerzellen; Bioreaktoren zur Herstellung von Stoffwechselproduckten solcher Zellen; Bioreaktoren zur Herstellung von Peptiden oder Proteinen, welche von solchen Zellen exprimiert werden; Bioreaktoren zur Energiegewinnung mit Hilfe solcher Zellen; Bioreaktoren zur Vermehrung von Viren mit Hilfe solcher Zellen; Bioreaktoren für den Abbau von Stoffen mit Hilfe solcher Zellen; Bioreaktoren zur Herstellung von Nahrungsmitteln mit Hilfe solcher Zellen; Bioreaktoren zur Erzeugung von Biogas mit Hilfe solcher Zellen; und Kombinationen davon.

Eine *Einzelsteuereinheit* 230 (wie weiter unten mit Bezug auf **Figur 2** beschrieben) ist ein technisches Bauteil, bei welchem es sich um einen Sensor 140 A-N, ein Aktor bzw. Aktuator, eine Pumpe, ein Zählwerk bzw. einen Totalizer sowie jedes geeignete Prozessverarbeitungsgerät handeln kann. Insbesondere kann es sich bei einer Einzelsteuereinheit 230 auch um eine beliebige Kombination der o.g. technischen Bauteile, auch Steuerungsmodul genannt, handeln, die als eine Einheit agieren kann, wie z.B. eine Umwälzpumpe, einen Servo, ein Steuergerät oder einen Pneumatikzylinder mit Rückmeldung an eine Steuerungseinheit. Zudem kann sich eine Einzelsteuereinheit 230 aus einer Vielzahl von Einzelsteuerungseinheiten zusammensetzen, z.B. kann sich ein Hauptkontrollmodul aus einer Kombination einer Vielzahl von automatischen Ein/Aus-Ventilsteuerungsmodulen bzw. On/Off Automatic Block Valves zusammensetzen. So kann es sich bei Einzelsteuereinheiten 230 beispielsweise um
- ein Regulierungsgerät handeln, welches sich aus einer Sendeeinheit bzw. einem Transmitter, einer Regelungseinheit bzw. einem Controller und einem Regelventil bzw. einer Control Valve zusammensetzt;
- ein zustandsorientiertes Gerät handeln, welches ein automatisches Ein/Aus-Ventilsteuerungsmodul mit Positions-Rückmeldungsschaltern umfasst, das über einen Nennwert des Gerätes betrieben und/oder gesteuert werden kann; oder
- einen Header handeln, der eine Vielzahl von automatischen Ein/Aus-Ventilsteuerungsmodulen beinhaltet und der die Ventile derart koordiniert, dass ein Fluss durch die Ventile zu einem oder mehreren Zielen führt.

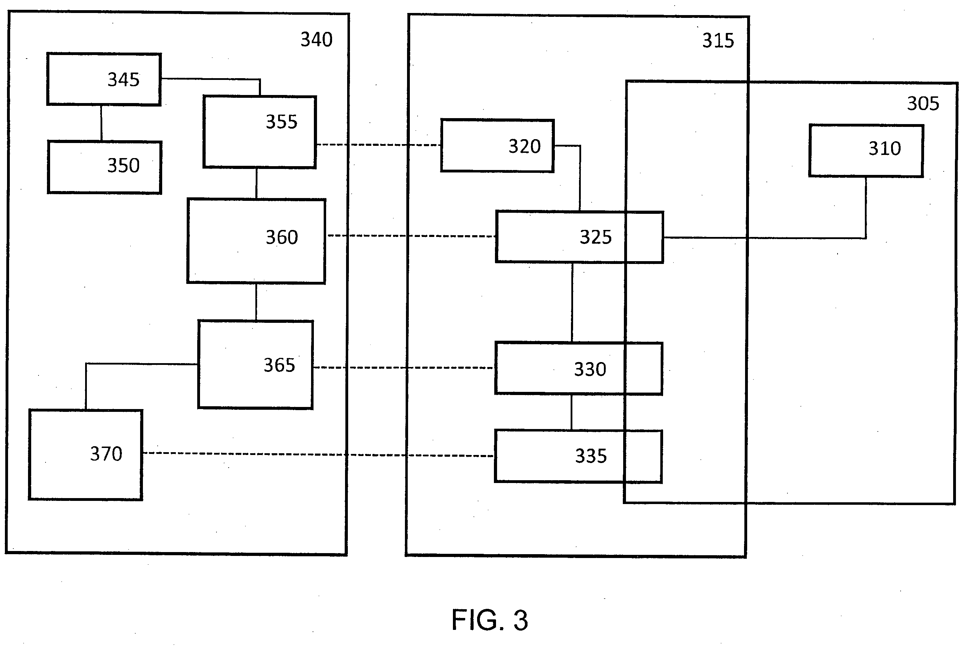

Ein *Kontrollmodul 325 bzw. Control Module* (wie weiter unten mit Bezug auf **Figur 3** beschrieben) umfasst insbesondere Variablen, die zu einer physikalisch gemessenen oder errechneten Größe zusammengehören. Optional kann das Kontrollmodul die Variablen auch steuern bzw. regeln. Es können unterschiedliche Typen von Kontrollmodulen bereitgestellt werden: Controller, Process Variable, Digital Variable und/oder Offline Variable.

Ein *Device* 205, 210, 215 (wie weiter unten mit Bezug auf **Figur 2** beschrieben) stellt insbesondere ein Interface bzw. Schnittstelle für die Ankopplung eines Elements an eine Einheit dar. Beispielsweise kann ein Sensor Device 205 ein Interface für die Ankopplung eines O₂/CO₂ Analysesensors an die Einheit darstellen. Ferner kann ein Reaktor Device 210 ein Interface bzw. Schnittstelle für die Ankopplung eines Bioreaktors 225 darstellen. Ebenso kann ein Einzelsteuereinheits-Device 215 ein Interface für eine Ankopplung einer Waage an die Einheit darstellen.

Ein *Batch* ist insbesondere eine Sammlung von Daten bzw. Datensätzen, die von den jeweiligen Komponenten einer Einheit innerhalb eines spezifischen, d.h. vorbestimmten bzw. vorbestimmbaren, Zeitraums als Messdaten erfasst werden. Insbesondere ist ein Batch eine Aufzeichnung von Messdaten über den Prozess, der von der jeweiligen Einheit durchgeführt wird und so auch archiviert werden kann. Jeder Batch kann nach Beendigung des Prozesses in einer Datenbank gespeichert werden. So kann dieser immer wieder zu einer Analyse und/oder Auswertung herangezogen werden. Somit beinhaltet jeder Batch immer wieder die charakteristisch und zeitlich gleichen Prozessverläufe. Die Daten bzw. Datensätze können zeitlich in der Reihenfolge, in der sie hintereinander zusammengefasst werden, gespeichert und über die Ausgabeeinheit angezeigt werden. Beispielsweise kann jeder Batch alle Messdaten beinhalten, die im Zeitraum zwischen einer Befüllung einer Einheit, z.B. eines Reaktors bzw. Bioreaktors, bis zu einer kompletten Entleerung dieser Einheit nach einer spezifischen, d.h. vorbestimmten bzw. vorbestimmbaren, Zeitspanne, z.B. einer Reaktionszeit bzw. Wachstumszeit, erfasst werden.

*Messdaten bzw. Prozessdaten* sind insbesondere Daten bzw. Datensätze, welche Informationen über einen Wert einer Eigenschaft zu einem gegebenen Zeitpunkt beinhalten. Messdaten können in einer Datenbank gespeichert werden.

Bei einem Stoff kann es sich insbesondere um eine Flüssigkeit, ein Gas und/oder einen Feststoff handeln. Der Stoff kann sich aus zumindest einer Komponente zusammensetzen, welche benötigt wird, um die Durchführung des Prozesses zu ermöglichen. So kann es beispielsweise für eine Kultivierung bestimmter Organismen, z.B. Zellen bzw. bestimmter Teile von Organismen, notwendig sein, wichtige Bedingungen, die ein optimales Wachstum der Organismen gewährleisten, zu schaffen und aufrechtzuerhalten. Beispielsweise kann es sich bei solchen wichtigen Bedingungen um eine Zusammensetzung eines Nährmediums bzw. einer Nährlösung bzw. eines Substrats, eine Sauerstoffzufuhr, einen pH-Wert und/oder eine Sterilität im Bioreaktor handeln. Zweck der Kultivierung kann eine Gewinnung von Organismen, z.B. Zellen und Teilen von Organismen sein. Ein weiterer Zweck der Kultivierung kann eine Gewinnung von Stoffwechselprodukten sein, welche als Wirkstoff in der pharmazeutischen Industrie und/oder als Grundchemikalie in der chemischen Industrie verwendet werden. Ferner kann es sich beim Zweck um einen Abbau chemischer Verbindungen in z.B. Kläranlagen oder um eine Herstellung von Alkoholika handeln.

Die Messdaten 335 (wie weiter unten mit Bezug auf **Figur 3** beschrieben) können über die Anzeigeeinheit 100 beispielsweise in Form von Messkurven dargestellt werden. Alternativ und/oder zusätzlich können die Messdaten in Form von ein oder mehreren Diagrammen, z.B. Kreisdiagrammen, Liniendiagrammen, Balkendiagrammen, Organigrammen, Gantt-Diagrammen, Fluss-Diagrammen, Ursache-Wirkungs-Diagrammen und/oder Blockdiagrammen dargestellt werden.

Um eine Optimierung von Prozessen, beispielsweise einer Zellkultivierung, zu ermöglichen, ist es notwendig, einen genauen Überblick über wichtige Faktoren des Prozesses, z.B. einen Sauerstoffgehalt, zu haben. Ferner ist es wichtig, Erkenntnisse über Auswirkung einer Änderung dieser Faktoren auf den Prozesserfolg, z.B. eine Auswirkung auf das Zellwachstum durch eine Sauerstoffzufuhr bzw. durch eine Zugabe eines neuen Nährmediums, zu erhalten. Dies kann auf effiziente Weise, z.B. bei zwei gerade laufenden Prozessen in unterschiedlichen Einheiten 135 A-N, realisiert werden, indem Messdaten der Sensoren 140 A-N der zwei Einheiten 135 A-N in einer zeitlichen Korrelation dargestellt werden, welche Aufschlüsse über einen Zusammenhang der dargestellten Messdaten ermöglicht. Die einzelnen Komponenten des Computersystems 160 können über ein geeignetes Netzwerk 145, z.B. ein "local area network" (LAN) oder ein "wide area network" (WAN), miteinander verbunden werden.

Optional kann das Computersystem 160 eine Erfassungseinheit 150 umfassen, die eingerichtet ist, die zeitliche Korrelation automatisch zu erkennen. Beispielsweise kann die Erfassungseinheit 150 einen Verlauf der Messdaten der jeweiligen Einheiten 135 A-N miteinander vergleichen und Messdaten, die sich entsprechen, unabhängig von einer absoluten Zeit ihrer Erfassung in zeitlich entsprechender Weise über die Anzeigeeinheit 100 in den jeweiligen Anzeigebereichen 105, 110 anzeigen.

Eine zeitliche Korrelation der Messdaten kann auf eine beliebige Art und Weise, stark in Abhängigkeit der jeweiligen Anwendung und deren Aufgabe oder Fragestellung, erkannt werden. Beispielsweise können Messdaten unterschiedlicher Einheiten
- einen oder mehrere spezifische, d.h. vorbestimmte bzw. vorbestimmbare, absolute Werte bzw. Schwellenwerte und/oder einen oder mehrere relative Werte, z.B. einen pH-Wert, einen CO₂-Wert oder einen O₂-Wert, über- oder unterschreiten;
- einem gemessenen Höchststand bzw. Tiefststand entsprechen;
- einem spezifischen, d.h. vorbestimmten bzw. vorbestimmbaren, Kurvenanstieg, welcher z.B. eine Wachstumsrate darstellen kann, entsprechen;
- einem spezifischen, d.h. vorbestimmten bzw. vorbestimmbaren, Kurvenabstieg, welcher z.B. eine Sterberate darstellen kann, entsprechen;
- einem Alarm entsprechen, welcher ausgelöst werden kann, wenn die für den jeweiligen biologischen, biochemischen, chemischen und/oder physikalischen Prozess wichtigen Bedingungen über- bzw. unterschritten werden, z.B. bei einem zu geringen oder zu hohem Sauerstoffgehalt und/oder pH-Wert in der Materie, die sich im Reaktor befindet;
- nach Durchführung einer deskriptiven univariaten Datenanalyse (z.B. eine Prüfung einer Verteilung, eine Berechnung von Verteilungskenngrößen, eine grafischen Datenanalyse oder eine Korrelationsanalyse) einer spezifischen, d.h. vorbestimmten bzw. vorbestimmbaren, Korrelation zwischen einzelnen Variablen der Messdaten entsprechen;
- nach Durchführung einer Hauptkomponentenanalyse bzw. Principal Component Analysis (PCA) eine spezifische, d.h. vorbestimmte bzw. vorbestimmbare, Datendichte aufweisen;
- nach Durchführung einer Partial Least Squares Regression (PLS) und/oder einer Diskriminanzanalyse und/oder Clusteranalyse spezifische, d.h. vorbestimmte bzw. vorbestimmbare, Strukturen oder Klassen innerhalb der Daten aufweisen; und/oder
- nach Durchführung eines Verfahrens der erweiterten multivariaten Datenanalyse (z.B. Multiway Principal Component Analysis) spezifische, d.h. vorbestimmte bzw. vorbestimmbare, Strukturen in den Daten aufweisen.

Alternativ oder zusätzlich kann ein Benutzer mithilfe einer geeigneten Eingabevorrichtung, wie z.B. einer Tastatur bzw. eines Keyboards und/oder einer Computermaus, in einem angezeigten Messdatenverlauf einen Marker setzen. Auf diesen Marker hin kann eine zeitliche Korrelation der Messdaten erkannt werden.

Alternativ oder zusätzlich kann jeder Batch in verschiedene Phasen unterteilt werden. Beispielsweise kann eine erste Phase einem Einfüllen einer Materie in eine Einheit entsprechen, eine zweite Phase einem Zufügen von Zellen zu der Materie, eine dritte Phase einem Zufügen weiterer Zellen zu der Materie etc. entsprechen. In diesem Fall kann eine zeitliche Korrelation der Messdaten erkannt werden, indem Messdaten unterschiedlicher Einheiten einem Beginn einer bestimmten Phase entsprechen.

Dies hat den Vorteil, dass Messdaten der jeweiligen Einheiten 135 A-N, welche nach einem automatischen Vergleich eine spezifische, d.h. vorbestimmte bzw. vorbestimmbare, Bedingung erfüllen, unabhängig von einer absoluten Zeit ihrer Erfassung automatisch über die Anzeigeeinheit 100 dargestellt werden können, so dass Aufschlüsse über einen Zusammenhang dieser Daten ermöglicht werden.

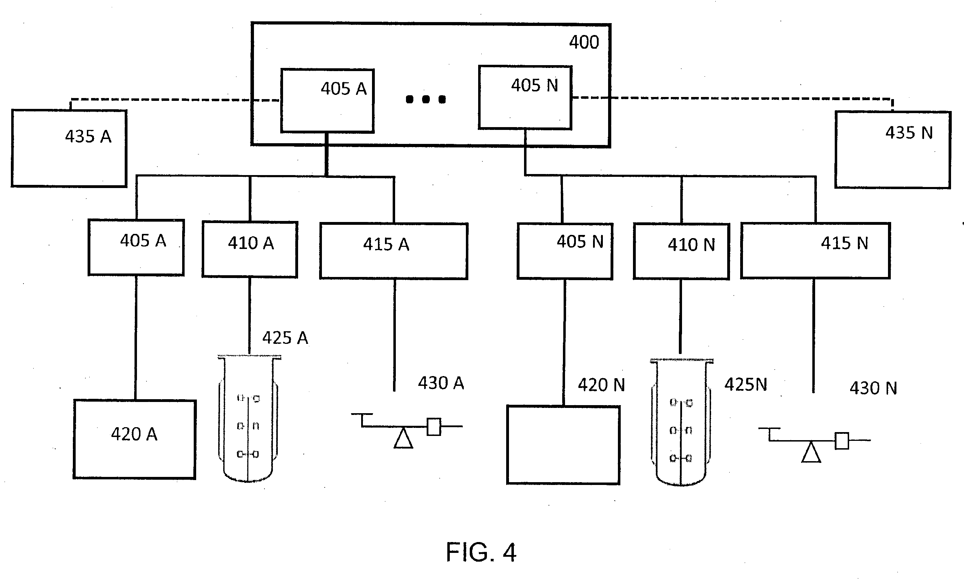

Optional kann das Computersystem 160 ein Steuerungsmodul 155 umfassen, welches eingerichtet ist, zumindest zwei Einheiten 135 A-N zu einer Gruppe zusammenzufassen, so dass die Gruppe gleichzeitig über das Steuerungsmodul 155 gesteuert werden kann (wie weiter unten mit Bezug auf **Figur 4** erläutert).

Das Computersystem 160 hat insbesondere den Vorteil, dass Auswirkungen unterschiedlicher wichtiger Prozessfaktoren bzw. einer Änderung ein oder mehrerer solcher Prozessfaktoren auf eine einfache und effiziente Weise in Echtzeit erfasst werden können. Somit wird eine kontinuierliche Optimierung bzw. Verbesserung der Prozessabläufe ermöglicht. Insbesondere wird dadurch eine verbesserte, kontinuierliche Benutzer-Maschinen-Interaktion erzielt, da eine Einstellung von biologischen, chemischen, biochemischen und/oder physikalischen Prozessparametern während laufender Prozesse in den jeweiligen Einheiten 135 A-N gezielt und in Abhängigkeit des entsprechenden Zustands des Prozesses eingestellt bzw. eingegeben werden können. Dadurch wird eine Koordinierung bzw. Abstimmung der technischen Abläufe in den jeweiligen Einheiten 135 A-N ermöglicht.

Darüber hinaus wird über die Anzeigeeinheit 100 eine Anzeige unterschiedlicher technischer Zustände, die in den jeweiligen Einheiten 135 A-N herrschen, ermöglicht.

Insbesondere kann über die Anzeigeeinheit 100 ein Alarm angezeigt werden, wenn die für den jeweiligen biologischen, biochemischen, chemischen und/oder physikalischen Prozess wichtigen Bedingungen über- bzw. unterschritten werden, z.B. bei einem zu geringen oder zu hohem Sauerstoffgehalt und/oder pH-Wert in der Materie, die sich im Reaktor befindet.

Zudem wird eine technische Steuerung der jeweiligen Einheiten 135 A-N ermöglicht, indem eine Einstellung von für den jeweiligen Prozess wesentlicher Parameter während der jeweiligen Prozessabläufe in Echtzeit ermöglicht wird.

Ein weiterer Vorteil liegt darin, dass über die zeitliche Korrelation relevante technische Zusammenhänge der Prozesse in den jeweiligen Einheiten 135 A-N unabhängig von einer absoluten Zeit der erfassten Messdaten über die Anzeigeeinheit 100 zugänglich gemacht werden können. Darüber hinaus kann die zeitliche Korrelation über die Dauer der Prozesse in den jeweiligen Einheiten 135 A-N aufrechterhalten bleiben.

Somit wird es einem Benutzer ermöglicht, die technischen Aufgaben der Überwachung von technischen Parametern, Steuerung der technischen Prozessparameter, Erfassung technischer Zusammenhänge der verschiedene Prozesse sowie Überwachung der Auswirkungen verschiedener technischer Parameter auf einen Prozessablauf, in den jeweiligen Einheiten 135 A-N effizienter und schneller zu bewältigen.

Figur 2 zeigt ein Blockdiagramm einer beispielhaften Einheit 200. Insbesondere umfasst die Einheit 200 einen Reaktor 225 oder einen Filter, der eingerichtet ist, einen Stoff bzw. eine Materie aufzunehmen, um an diesem Stoff zumindest einen biologischen, biochemischen, chemischen und/oder physikalischen Prozess durchzuführen. Zudem umfasst die Einheit 200 einen Sensor 220, z.B. einen O₂/CO₂ Analysator, der eingerichtet ist, Messdaten betreffend den Prozess zu erfassen. Darüber hinaus umfasst die Einheit 200 eine Einzelsteuereinheit 230, z.B. eine Waage und/oder ein oder mehrere interne Einzelsteuereinheiten 235. Jede der internen Einzelsteuereinheiten 235 kann eingerichtet sein, Berechnungen über die erfassten Messdaten durchzuführen und/oder Proben des Stoffes, an dem der Prozess durchgeführt wird, zu entnehmen und/oder zu analysieren.

Jede dieser Komponenten kann über ein entsprechendes Device 205, 210, 215 an die Einheit 200 gekoppelt sein. Jedes Device 205, 210, 215 kann eine Kommunikationsschnittstelle bzw. Communication Interface sein. Die Trennung zwischen Einheiten 200 und Devices 205, 210, 215 hat den Vorteil, dass eine Konfiguration einer Einheit 200 auf einfache und effiziente Weise durchgeführt werden kann, da die jeweiligen Komponenten 220, 225, 230 mithilfe der Devices 205, 210, 215 an die Einheit 200 angebunden werden können. Insbesondere ist das O₂/CO₂-Analysegerät 220 über eine Universal Serial Bus (USB)-Verbindung an die Einheit 200 gekoppelt, wobei ein Sensor Device 205 als Kommunikationsschnittstelle dient. Der Bioreaktor 225 kann beispielsweise via Ethernet entsprechende Messdaten an die Einheit 200 senden und/oder weitere Daten mit der Einheit 200 austauschen, wobei ein Reaktor-Device 210 hierzu als Kommunikationsschnittstelle dient. Die Waage 230 kann mit einem Einzelsteuereinheit-Device 215 an die Einheit 200 gekoppelt werden. Optional können benötigte interne Einzelsteuereinheiten 235 an die Einheit 200 gekoppelt werden.

Ein *Kontrollmodul* 325 *bzw. Control Module* (wie weiter unten mit Bezug auf **Figur 3** **beschrieben**) kann Variablen, die zu einer physikalisch durch den Sensor 220 gemessenen oder errechneten bzw. ermittelten Größe zusammengehören, zusammenfassen. Optional kann das Kontrollmodul 325 die Variablen auch steuern bzw. regeln. Es können unterschiedliche Typen von Kontrollmodulen 325 bereitgestellt werden: Controller, Process Variable, Digital Variable und Offline Variable. Beispielsweise kann die Einheit 200 einen Temperatursensor für die Erfassung einer Temperatur beinhalten. Dem Temperatursensor entsprechend kann ein Kontrollmodul 325 bereitgestellt werden. Dieses Kontrollmodul 325 kann vom Typ Controller sein. Im vorliegenden Beispiel kann ein Kontrollmodul 325 folgende Variablen aufweisen:
- Temp.Value → Istwert Temperatur;
- Temp.Setpoint → Sollwert Temperatur;
- Temp.Output → Stellgröße Temperatur;
- Temp.Mode → Modus Temperatur, z.B. Automatic, Manual, Profile, Cascade;
- Temp.Status → Status Temperatur, z.B. Remote, Off. Der Statuswert kann angeben, ob der Wert schreibbar ist. Der Statuswert stellt eine Sicherheitsfunktion dar;
- Temp.Value.EngineeringUnit → °C;
- Temp.Output.EngineeringUnit → %;
- Temp.HighLimit → Gibt einen oberen Temperaturschwellenwert an; kann bei Überschreitung eines spezifischen, d.h. vorbestimmten bzw. vorbestimmbaren, Wertes eine Warnung ausgeben;
- Temp.HighHighLimit → Löst bei Überschreitung einer absoluten Temperaturobergrenze eine Alarm aus;
- Temp.LowLimit → Gibt eine unteren Temperaturschwellenwert an; kann bei Unterschreitung eines spezifischen, d.h. vorbestimmten bzw. vorbestimmbaren, Wertes eine Warnung ausgeben;
- TempLowLowLimit → Löst bei Unterschreitung einer absoluten Temperaturuntergrenze einen Alarm aus;
- Temp.UnitID → Gibt die ID der Einheit 200 an, auf der das Kontrollmodul 325 angelegt ist. Somit kann jedes Kontrollmodul 325 eindeutig zugeordnet werden;

Jeder Kontrollmodul-Variablen kann eine eigene MappingShortID zugeordnet werden. Dies hat den Vorteil, dass die entsprechend einlaufen Messdaten eindeutig einer Kontrollmodulvariablen zugeordnet werden können.

**Figur 3** zeigt einen Überblick über eine beispielhafte Struktur und Zusammenhänge von Messdaten 335 einer Einheit 320, die zu einem Batch 350 zusammengefasst werden.

Aus Kommunikationssicht 305 kann dabei eine Einrichtung 310 mit jeder Einheit 320 über ein Kontrollmodul 325 kommunizieren. Jedes Kontrollmodul 325 kann Kontrollmodulvariablen 330 umfassen. Ein Kontrollmodul kann eine Zusammenfassung von Variablen und/oder Werten sein, die zu einer physikalischen Größe gehören. Jedes Kontrollmodul hat zumindest einen Wert (z.B. 34,5), eine Einheit. (z.B. °C), eine Unit und eine "Source" (eine Vorrichtung aus der die Werte bzw. eine Berechnung der Werte stammen). Optional kann ein Kontrollmodul zusätzliche Variablen enthalten (z.B. Sollwert, Stellgröße und/oder LowLimit bzw. Alarmgrenze). Zudem ist ein Blick auf die Messdaten 335 möglich. Aus Anwendersicht 315 bzw. Monitoring-Sicht ist jedes Kontrollmodul 325 einer Einheit 320 zugeordnet und kann ein oder mehrere Kontrollmodulvariablen 330 aufweisen. Im Trend können Kontrollmodul-Variablen angezeigt werden, jedoch nicht alle, sondern nur die vom Typ Istwert, Sollwert und/oder Stellgröße. Jeder dieser Kontrollmodulvariablen 330 können ein oder mehrere Messdaten 335 zugeordnet werden. Die Anwendersicht zeigt eine aktuell gültige Konfiguration einer Einheit 320 auf. Ein Kontrollmodul kann je nach Typ mehrere Variablen aufweisen. Jeder Variablen kann aber nur ein Messdatum/Messwert zugeordnet werden.

Aus einer historischen Batch-Messdaten-Sicht bzw. Historical Batch Data-Sicht 340 werden die Messdaten über die Batch-Prozesse jeder Einheit 320 im Batch 345 gespeichert (Messdatenspeicherung 370). Jedem Batch 345 wird dabei eine Start- und Stoppzeit 350 zugeordnet. Die Batch Startzeit entspricht dem Aufzeichnungs-Start und gleichzeitig dem dazugehörigen biologischen/chemischen Prozessstart an der Einheit. Die Batch-Stoppzeit entspricht einem entsprechenden biologischen/chemischen Prozessende und gleichzeitig dem Aufzeichnungsende. Auch die während des Batch-Starts konfigurierten Kontrollmodule 325, deren Kontrollmodulvariablen 330 sowie dessen während des Prozesses auflaufende Messdaten 335 werden entsprechend im Batch 345 hinterlegt. Somit hinterlegt jeder Batch 345 einen Schnappschuss der aktuell gültigen Konfiguration sowie aller Messdaten bzw. Prozessdaten.

Dies hat den Vorteil, das abgeschlossene/historische Batches zusammen mit deren zu ihrer Zeit gültigen Konfiguration im Analyse Bereich betrachtet bzw. analysiert werden können. Gleichzeitig ermöglicht das Computersystem 160 über die Administration einer bereits neue/andere/geänderte Konfiguration (neue oder geänderte Geräte an der Einheit, umbenannte Einheit, geänderte ControlModule Namen, gelöschte Einheit) der jeweiligen Einrichtungen bzw. Devices 205, 210, 215. Über die Anwender- bzw. Monitoring-Sicht wird ein Monitoring der Messdaten bzw. Prozessdaten zu einer Einheit 320 ermöglicht. Mittels der Historical Batch Data Sicht wird eine Archivierung der Messdaten und/oder eine Analyse, insbesondere einer Offline-Analyse dieser ermöglicht. Die Kommunikationssicht ermöglicht eine Anzeige eines Messdaten-Flusses sowie eine Zuordnung der Messdaten.
Die Batches 345 und deren zugehörigen Prozessdaten können in einer Datenbank gespeichert werden. Diese Daten können zur Verwendung in anderen Tools als CSV (Comma-separated values)-Files aus der SQL-Datenbank exportiert werden. Vorzugsweise kann in der Datenbank eine effiziente Datenkomprimierung implementiert sein. Dies hat den Vorteil, dass die gespeicherten Messdaten schnell und in sehr kurzen, zeitlichen Abständen auf einen entsprechenden Datenbankaufruf hin ausgegeben werden können.

Beispielsweise kann sich ein Messdatum aus folgenden Variablen zusammensetzen:
- MappingShortID → Identifizierung Kontrollmodul-Variable;
- Timestamp → Zeitstempel: Wann ist das Messdatum in der Einheit 320 eingegangen. Der Zeitstempel kann vom Computersystem 160 für jedes Messdatum zentral vergeben werden, um eine optimale Genauigkeit zu erzielen;
- Value → Messwert des Messdatums;
- Quality → binärer Wert, z.B. 0 für "good" und 1 für "bad"; die Qualität kann ausschlaggebend dafür sein, ob das Messdatum über die Anzeigeeinheit 100 angezeigt wird;
- Checksum bzw. Prüfsumme → Wird vom Computersystem 160 generiert, um eine Datenmanipulation zu verhindern. Dies hat den Vorteil, dass die Sicherheit im Computersystem 160 erhöht wird.

Beispielsweise kann eine Erfassungseinheit 150 (wie oben mit Bezug auf Figur 1 beschrieben) im Computersystem 160 die zeitliche Korrelation anzeigen, indem zwei Einheiten 135 A-N zu einer Gruppe 400 zusammengefasst werden (Unit Grouping).

**Figur 4** zeigt ein Beispiel einer Gruppe 400 zumindest zweier Einheiten 405 A-N. Bei einer Gruppierung ist es unerheblich, ob die Einheiten 405 A-N bzw. Units vom gleichen Typ sind bzw. dieselben Sensoren 420 A-N bzw. Einzelsteuereinheiten 430A-N besitzen.

Somit ermöglicht das System eine freie Unit Grouping-Kombination, von welcher die Erfassungseinheit 150 die zeitliche Korrelation anzeigen kann. Die Anbindung der jeweiligen Komponenten an die jeweiligen Einheiten 405 A-N erfolgt dabei analog zur Anbindung wie weiter oben mit Bezug auf **Figur 2** beschrieben, d.h. die Sensoren 420 A-N werden mithilfe eines jeweiligen Sensor Devices 406 A-N an die jeweilige Einheit 405 A-N angebunden. Die Reaktoren 410 A-N können mithilfe entsprechender Reaktor-Devices 410 A-N an die jeweilige Einheit 405 A-N gekoppelt werden. Die Einzelsteuereinheiten 430 A-N können mithilfe entsprechender Einzelsteuer-Devices 415 AN an die jeweilige Einheit 405 A-N gekoppelt werden. Optional können die jeweiligen internen Einzelsteuereinheiten 435 A-N an die jeweiligen Einheiten 405 A-N gekoppelt werden.

Die Messdaten der jeweiligen Batches können dabei mithilfe der Anzeigeeinheit 100 entweder in einem gemeinsamen Anzeigebereich oder optional in jeweils eigenen Anzeigebereichen über die Anzeigeeinheit 100 ausgegeben werden. Für den Fall, dass das Computersystem 160 drei oder mehr Einheiten 405 A-N umfasst, kann eine Gruppe 400 aus einer beliebig wählbaren Kombination der Einheiten 405 A-N, welche zeitlich korreliert werden sollen, erstellt werden. Zudem ist es möglich, dass unterschiedliche Benutzer mithilfe jeweils einer Anzeigeeinheit 100 individuell eine beliebige Gruppe 400 erstellen können, so dass eine individuelle zeitliche Korrelation von den der Gruppe 400 entsprechenden Messdaten initiiert bzw. durchgeführt werden kann. Zudem hat jeder Benutzer die Möglichkeit, einen Schnappschuss der Messdaten, die über die Anzeigeeinheit 100 entsprechend zeitlich korreliert dargestellt werden, durch Eingabe eines entsprechenden Befehls, z.B. über eine Aktivierung eines "Report"-Buttons, zu erstellen und zu hinterlegen, so dass er jederzeit auf die zeitlich korrelierten Daten zugreifen kann. Jede Gruppe 400 kann auch während laufender Prozesse automatisch, z.B. indem eine spezifische, d.h. vorbestimmte bzw. vorbestimmbare, Bedingung erfüllt wird, oder vom Benutzer manuell wieder aufgelöst werden.

Beim Unit Grouping werden Messdaten des jeweiligen Sensors über den jeweiligen Prozess des Reaktors und/oder Filters sowie optional die jeweiligen zusätzlichen Einzelsteuereinheiten und internen Einzelsteuereinheiten zu jeweils einem eigenen Batch zusammengefasst und über die Anzeigeeinheit 100 angezeigt.

Da es ab einer bestimmten Dichte von Daten bzw. Datensätzen nicht möglich ist, alle Daten, die in einem Batch zusammengefasst sind, gleichzeitig über die Anzeigeeinheit 100 anzuzeigen, kann das Computersystem 160 eine Voreinstellung umfassen, nach welcher die Anzeigeeinheit 100 immer die letzten, neuesten N Daten bzw. Datensätze, die erfasst und zum Batch hinzugefügt wurden, angezeigt werden, wobei N eine spezifische, d.h. vorbestimmte bzw. vorbestimmbare, Anzahl von Daten bzw. Datensätzen definiert.

Die zeitliche Korrelation kann angezeigt werden, indem ein Zeitfenster spezifischer, d.h. vorbestimmter bzw. vorbestimmbarer, Größe derart auf die Messdaten der jeweiligen Einheiten 405 A-N angewandt wird, dass Messdaten, die sich im Zeitfenster befinden, unabhängig von einer absoluten Zeit ihrer Erfassung zeitlich korreliert dargestellt werden. Diese zeitliche Korrelation bzw. Synchronisation der Messdaten kann dabei in der Anzeigeeinheit 100 erhalten bleiben, auch wenn neue Messdaten im Batch aufgenommen werden. Die Anzeigeeinheit 100 kann die zeitliche Korrelation der Daten, die sich innerhalb des Zeitfensters befinden, darstellen. Insbesondere handelt es sich bei der Synchronisation um eine besondere Art der zeitlichen Korrelation, da Messdaten, die in einem Anzeigebereich bzw. Anzeigefenster in ihrer Größe im Verhältnis zu Messdaten, die in einem zweiten Anzeigebereich bzw. Anzeigefenster angezeigt werden, verändert, insbesondere gestaucht bzw. gedehnt werden.

Dies hat den Vorteil, dass Messdaten, die von den Sensoren der zwei Einheiten erfasst und zeitlich korreliert wurden, unabhängig von der absoluten Zeit ihrer Erfassung über das Zeitfenster angezeigt werden. In diesem Fall werden die neuen Daten bzw. Datensätze, die von den Sensoren erfasst und zum entsprechenden Batch hinzugefügt werden, nicht über die Anzeigeeinheit 100 dargestellt.
In einem anderen Beispiel kann die zeitliche Korrelation angezeigt werden, indem Messdaten, die von Sensoren der jeweiligen Einheiten 405 A-N in unterschiedlichen zeitlichen Abständen gemessen wurden, in zeitliche Korrelation miteinander gebracht werden. Insbesondere kann die Größe des Zeitfensters, welche auf den jeweiligen Batch angewandt wird, derart angepasst werden, dass sich die Messdaten in ihren zeitlichen Abständen entsprechen. Optional kann die zeitliche Korrelation von einem Benutzer manuell eingestellt werden.

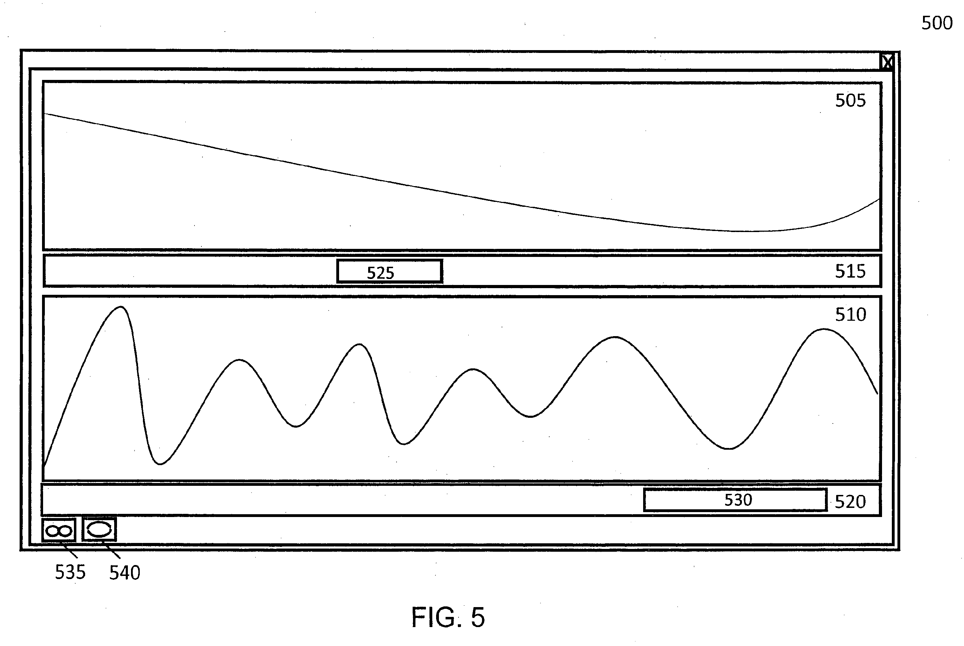

**Figur 5** zeigt ein eine exemplarische, vereinfacht dargestellte Anzeige einer Anzeigeeinheit 500, bei welcher im oberen Anzeigebereich 505 ein erster Schieberegler 525 vorhanden ist, der über eine erste Zoombar 515 verschoben werden kann. Dadurch kann ein Zeitfenster auf die Messdaten 335 eines ersten Batches, im Folgenden auch Trend genannt, angewandt werden. Da der erste Schieberegler 525 nicht an der rechten Kante anliegt, zeigt der obere Trend die Messdaten des Batches vom gewählten Zeitfenster aus an, unabhängig von einer absoluten Zeit der Erfassung der Messdaten 335. Im zweiten Anzeigebereich 510 werden Messdaten 335 einer anderen Einheit angezeigt, wobei ein zweiter Schieberegler 530 über eine zweite Zoombar 520 verschoben werden kann. In diesem Fall ist eine Synchronisation der Messdaten beider Einheiten nicht möglich, da die gesamte Zeitspanne der beiden Trends zu unterschiedlich ist.

Ein Benutzer kann eine Breite des Zeitfensters, d.h. eine Anzahl an anzuzeigenden Messdaten 335, steuern, indem er mithilfe einer geeigneten Eingabevorrichtung einen Punkt der Zoombar 515, 520, der sich außerhalb des Thumbs befindet, ansteuert. So kann jenes Ende des Thumbs, welches sich auf der Seite des angesteuerten Punktes befindet, zum Punkt hin erweitert werden.

Insbesondere kann ein Benutzer mithilfe einer geeigneten Eingabevorrichtung die Breite des Thumbs vergrößern oder verkleinern sowie den Thumb innerhalb der Zoombar 515, 520 beliebig nach rechts und links bewegen.

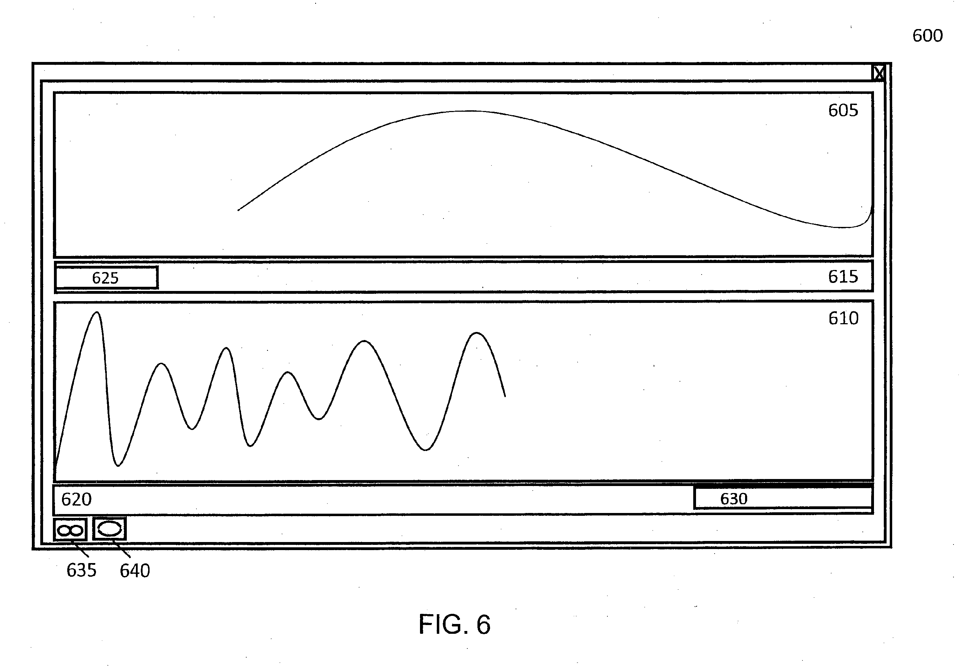

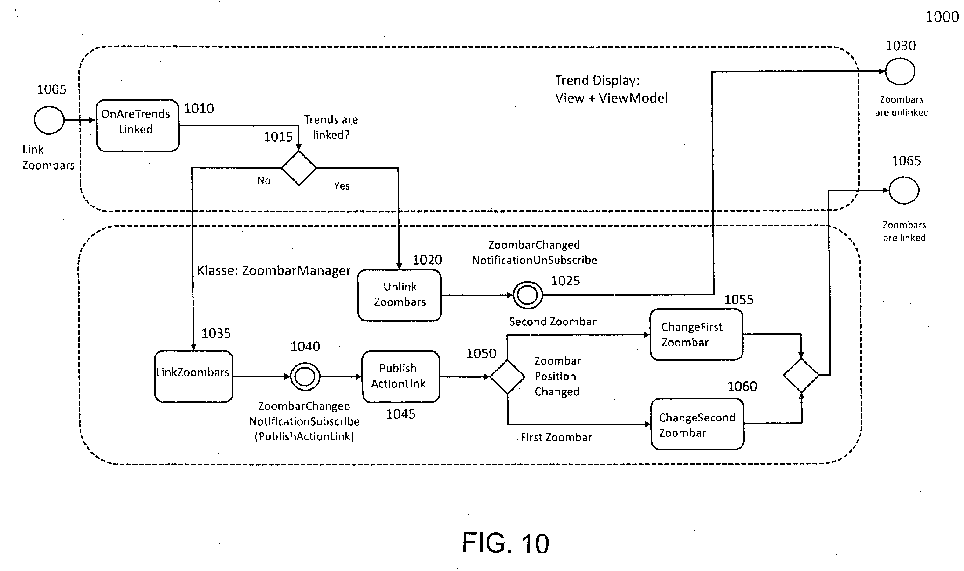

**Figur 6** zeigt ein Beispiel einer nur teilweise möglichen Aktion. Insbesondere zeigt Figur 6 über die Anzeigeeinheit 600 in einem ersten Anzeigebereich 605 einen ersten Messdatenverlauf bzw. Trend. Der Schieberegler 625 befindet sich ganz links in der Zoombar 615. Dies kann beispielsweise bedeuten, dass die ersten Messdaten 335 des entsprechenden Batches 345 angezeigt werden. In einem zweiten Anzeigebereich 610 wird ein zweiter Messdatenverlauf angezeigt, wobei sich der Schieberegler 630 ganz rechts von der entsprechenden Zoombar 620 befindet. Die beiden Trends können mithilfe eines entsprechenden Buttons 635 miteinander verlinkt werden. In diesem Fall ist es möglich, eine Aktion durchzuführen, die eine Verkleinerung des Bereichs durch Verkleinerung der jeweiligen Schieberegler 625, 630 beinhaltet. Allerdings wäre ein Verschieben des unteren Schiebereglers 630 (Aktor) nach links nicht möglich, da der obere Schieberegler 625 (Reaktor) bereits am linken Rand der zweiten Zoombar 610 anliegt. Eine beispielhafte Implementierung dieser Funktionalität wird weiter unten mit Bezug auf **Figur 10** beschrieben.

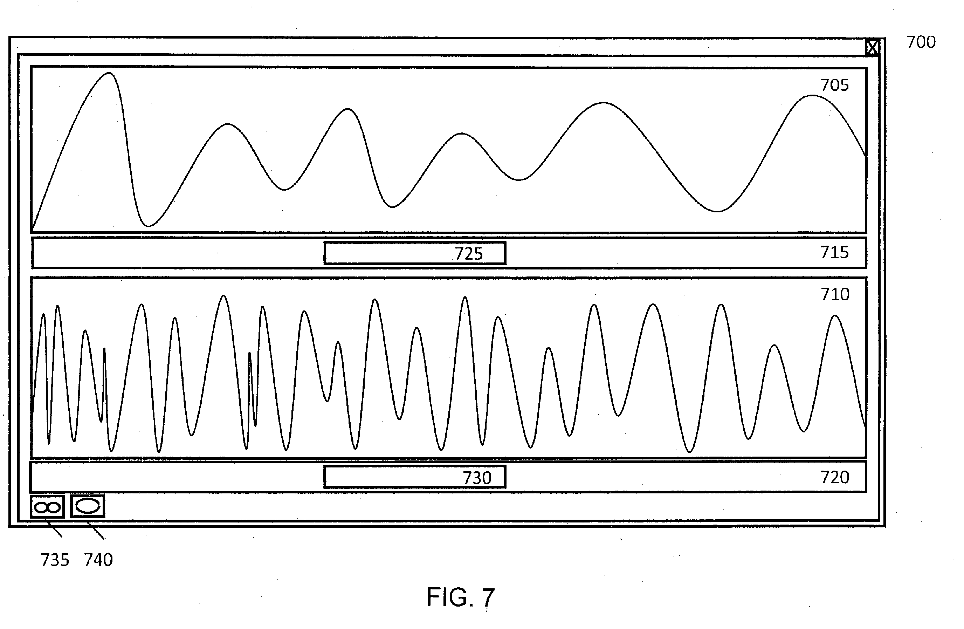

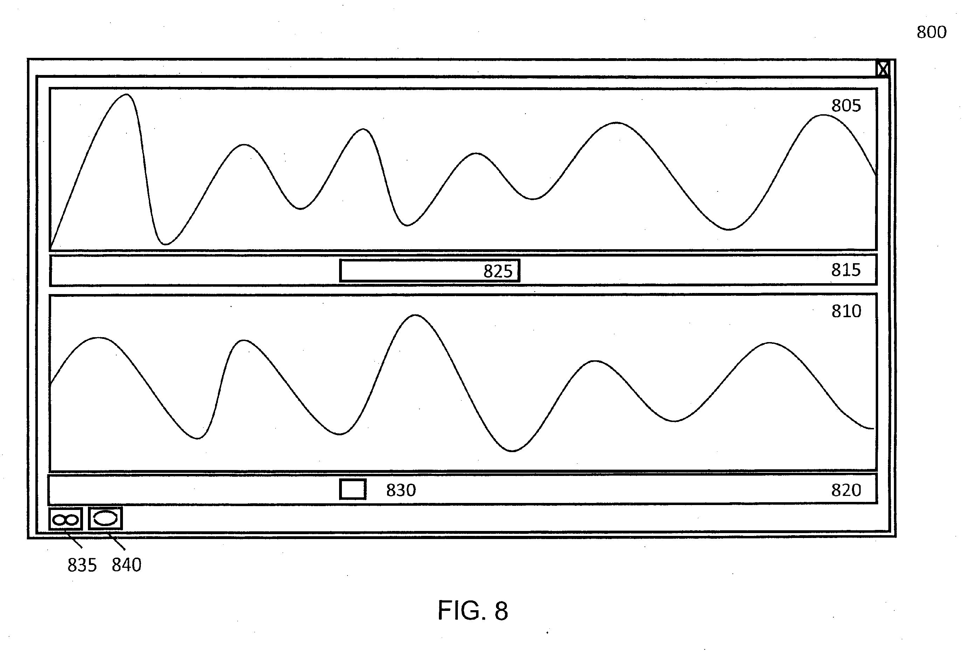

**Figur 7** zeigt zwei Messdatenverläufe bzw., bei denen eine Synchronisation der Zeitachse möglich ist, wobei **Figur 8** das Ergebnis der Synchronisation der Zeitachsen aufzeigt.

Insbesondere zeigt Figur 7 eine Anzeigeeinheit 700, die einen ersten Messdatenverlauf einer ersten Einheit 135 A-N, 200, 320 in einem ersten Anzeigebereich 705 sowie einen zweiten Messdatenverlauf einer zweiten Einheit 135 A-N, 200, 320 in einem zweiten Anzeigebereich 710 anzeigt. Die jeweiligen Schieberegler 725, 730 weisen dieselbe Größe auf und befinden sich im selben Bereich der jeweiligen Zoombar 715, 720.

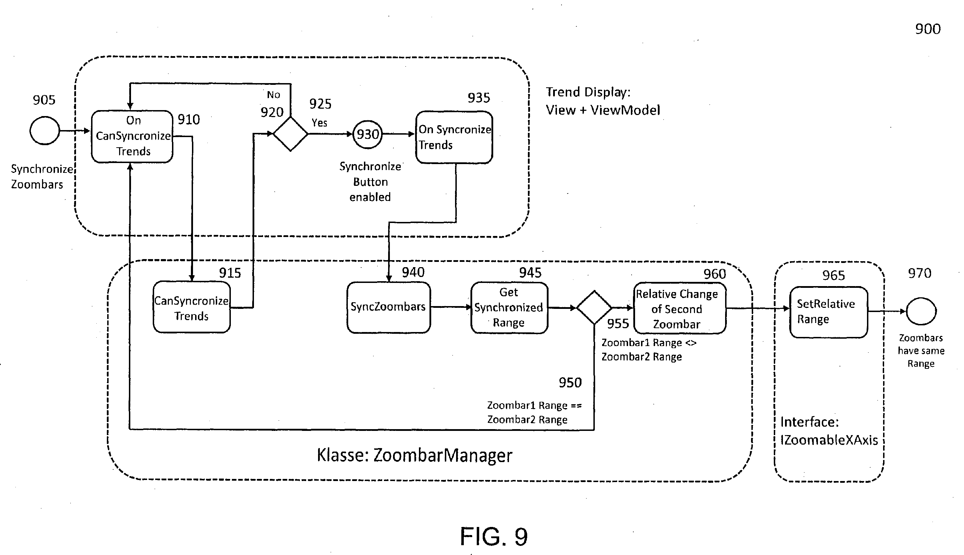

Die beiden Messdatenverläufe können durch Aktivierung eines entsprechenden Buttons 740 synchronisiert werden. Die technische Realisierung wird weiter unten mit Bezug auf **Figur 9** erläutert.

**Figur 8** zeigt die beiden Messdatenverläufe wie in **Figur 7**, nachdem die Synchronisation aufgrund einer Aktivierung des Buttons 740 (Figur 7) aktiviert wurde. Insbesondere werden die Ausschläge bzw. Änderungen der jeweiligen Messdaten in den jeweiligen Anzeigebereichen 805, 810 nun synchron zueinander, d.h. in einander gleichenden bzw. ähnelnden Abständen, angezeigt. Dabei hat sich die relative Größe des Schiebereglers der zweiten Anzeigeeinheit 830 verkleinert, so dass weniger Messdaten im entsprechenden Anzeigebereich 810 angezeigt werden, um die Synchronisierte Darstellung der Messdaten zu ermöglichen. Dabei kommt es darauf an, welcher Batch schon länger läuft. So ist es durchaus möglich, dass der untere oder der obere Trend bzw. dessen Zoombar verkleinert wird.

**Figur 9** zeigt ein Prozessflussdiagramm, welches einen Prozess der Synchronisierung der Zoombars 715, 720, 815, 820, wie weiter oben mit Bezug auf **Figuren 7** und **8** erläutert, visualisiert. Der Prozess wird mithilfe einer beispielhaften Implementierung der jeweiligen Methoden näher erläutert. Zunächst kann ein Benutzer mithilfe einer entsprechenden Aktivierung, z.B. durch Aktivierung des Buttons 740 in **Figur 7** bzw. 840 in **Figur 8**, im Folgenden auch "Synchronize"-Button genannt, die Synchronisierung zweier Zoombars 715, 720, 815, 820 starten 905 (Synchronize Zoombars). Beispielsweise kann der "Synchronize"-Button in einer WPF (Windows Presentation Foundation) via "Binding and ein Command "SynchTrendsCommand" gebunden werden, wie folgender beispielhafter Implementierung zu entnehmen ist:

```
     <Button x:Name="synchronizeTrendsButton"
                                  Width="40"
                                  Height="39"
                                  Command="{TemplateBinding syncTrend-
     scommand}"
                                  style="{DynamicResource
     SAF_Toolbar_Button}"
                                  self:FocusAttachments.SetFocusa-
     ble="True"
                                  IsTabstop="True"
                                  ToolTip="{Binding source={x:Static re-
     sources:Resource.Synchrom zeTrendButtonToolTip}}">
                              <ContentControl Margin="0,0,0,1"
                                             Content="CohtentControl"
                                              Focusable="False"
                                              IsTabstop="False"
                                              Style="{DynamicResource
     02_012_24_24_Sync}" />
                          </Button>
```

Ein Command "SynchTrendsCommand" kann in einer Klasse "TrendDisplay", d.h. einem Model View ViewModel (MWM) des Trends, implementiert sein. MWM ist eine Variante des Model View Controller-Musters (MVC), welches einer Trennung einer Darstellung und Logik einer Benutzerschnittstelle bzw. User Interface (UI) dient, und von der UI Plattform WPF unterstützt wird.

Eine Implementierung des Commands kann mithilfe zweier Methoden, "OnSynchronizeTrends" 935 und/oder "OnCanSynchronizeTrends" 910 erfolgen. Insbesondere kann die Methode OnSynchronizeTrends 935 in der Klasse "TrendDisplay" die Synchronisierung aufrufen. Die Methode "OnCanSynchronizeTrends" 910 hingegen kann überprüfen, ob eine Synchronisation möglich ist. Entsprechend kann der "Synchronized"-Button aktiviert bzw. enabled oder deaktiviert bzw. disabled werden:

```
     private void InitializeCommands()
          {
              this.ExpandzoombarCommand = new SAFCommand(this.onExpandTo-
    Max, this.CanExpandToMax);
              this.SyncTrendsCommand = new SAFCommand(this.OnSynchroni-
    zeTrends, this.OnCanSyncronizeTrends);
              this.LoadTemplateCommand = new SAFCommand(this.OnLoadTem-
     plate, this.CanLoadTemplate);
           }
```

Die Methode OnSynchronizeTrends 910 kann wiederum eine interne Methode "SyncZoombars" 940 der Klasse "ZoombarManager" mit beiden Zoombars als Übergabeparameter aufrufen:

```
     private void OnSynchromzeTrends(object obj)
           {
                this.zoombarManager.SyncZoombars(this.trendcontrol1.Zoom-
     Bar, this.trendControl2.ZoomBar);
           }
```

Ebenso kann die Methode "OnCanSynchronizeTrends" intern eine Methode "CanSyncZoombars" (nicht in **Figur 9** gezeigt) aus der Klasse "ZoombarManager" mit beiden Zoombars als Übergabeparameter aufrufen:

```
     private bool OnCanSyncronizeTrends(object obj)
           {
              return this.zoombarManager.canSynczoombars(this.trendcon-
     troll.zoomBar, this.trendcontrol2.ZoomBar);
          }
```

Im Folgenden wird eine beispielhafte Implementierung einer Feststellung, ob zwei Zoombars synchronisiert werden können, erläutert:
Insbesondere kann diese Feststellung implementiert werden, indem eine Methode "CanSyncZoombars" intern die Methode "GetSynchronizedRanges" aufruft, die auch beide Zoombars als Übergabeparameter übergibt. Insbesondere kann dabei als Rückgabewert ein Tuple einer X-Achse bzw. eines Zeitstrahls eines Batches und einer Zoombar-Range übergeben werden. Ist dieser Rückgabewert "Null", ist eine Synchronisierung nicht möglich, und der Button bleibt deaktiviert bzw. disabled. Beinhaltet der Rückgabewert ein Tuple entsprechend gültiger Objekte (X-Achse und Zoombar Range), findet eine Überprüfung statt, ob der minimale Bereich bzw. Range von der zu ändernden Zoombar kleiner ist als der Bereich, der gesetzt werden soll. Ist das der Fall, wird der Synchronize-Button aktiviert:

   ```
    public bool CanSyncZoombars(IZoomableXAxis zoomableXAxis1, Izoomab-
    lexAxis zoomablexAxis2)
          {
             var result = true;
             Tuple<IZoomablexAxis, zoombarRange> zoombarAndRange = GetSyn-
    cronizedRanges(zoomablexAxis1, zoomablexAxis2);
             if (zoombarAndRange != null)
             {
                 IZoomablexAxis zoombarToChange = zoombarAndRange.Item1;
                 ZoombarRange rangeToSet = zoombarAndRange.Item2;
                 if (zoombarToChange.MinimalThumbRange > rangeToSet.Maxi-
    mum - rangeToSet.Minimum) result = false;
             }
             else result = false;
             return result;
      }
```

Die Methode SyncZoombars 940 kann intern wiederum die Methode "GetSynchronizedRanges" 945 mit beiden Zoombars als Übergabeparameter aufrufen. Dabei kann im Vorfeld ein Flag "inSyncAction" gesetzt werden, um andere Prozesse bzw. Aktionen zwischenzeitlich zu blockieren. Rückgabewert der Methode kann wiederum ein Tuple (X-Achse und Zoombar Range) sein. Ist der Wert "Null", folgt keine Aktion. Beinhaltet das Tupel jeweils gültige Objekte, wird der Bereich der anzupassenden Zoombar mit den gelieferten Werten (relativ) gesetzt (SetRelativeRange 965; siehe **Figur 9**), d.h. Range Minimum und Range Maximum:

```
     public void Synczoombars(IZoomableXAxis zoomableXAxis1, IZoomableXAxis
     zoomablexAxis2)
          {
             inSyncAction = true;
             Tuple<IZoomableXAxis, zoombarRange> zoombarAndRange = GetSyn-
     cronizedRanges(zoomableXAxis1, zoomablexAxis2);
             if (zoombarAndRange != null)
             {
                 IZoomableXAxis zoombarTochange = zoombarAndRange.Item1;
                 ZoombarRange rangeToSet = zoombarAndRange.Item2;
                 zoombarTOchange.SetRelativeRange(rangeToSet.Minimum,
     rangeToSet.Maximum);
             }
              insyncAction = false;
          }
```

Dabei bezieht sich der relative Bereich auf den Trend, unabhängig von dessen absoluter Zeit. Dazu kann eine Methode "GetSynchronizedRanges" implementiert werden, welche die Bereiche bzw. Ranges aus den übergebenen X-Achsen aufruft:

```
     ZoombarRange zoombar1Range = zoomableXAxis1.GetRange();
     ZoombarRange zoombar2Range = zoomableXAxis2.GetRange();
```

Zudem kann eine Berechnung einer absoluten Zeitspanne erfolgen. Dies hat den Vorteil, dass Zoombars, die nicht gleich sind, mithilfe des Minimums bzw. Maximums der entsprechenden Bereiche verglichen werden:

```
           var zoombar1RangeSpan = (zoombar1Range.Maximum - zoom-
     bar1Range.Minimum) * (zoomablexAxis1.XAxisEnd - zoomableXAxis1.XAxis-
     Start);
           var zoombar2RangeSpan = (zoombar2Range.Maximum - zoom-
     bar2Range.Minimum) * (zoomableXAxis2.XAxisEnd - zoomableXAxis2.XAxis-
     Start);
```

In einem nächsten Schritt kann verglichen werden, welche Zeitspanne des jeweiligen Bereiches größer ist. Je nachdem muss die anzupassende Range anders berechnet werden:

```
     Range.Minimum = zoombar2Range.Minimum
     Range.Maximum = zoombar2Range.Minimum + RelativeChangeOfSecondZoom-
     bar(zoombar1Range.Maximum - zoombar1Range.Minimum, zoomableXAxis1,
     zoomablexAxis2)
```

Ein Range.Maximum kann mithilfe einer Methode RelativeChangeOfSecondZoombar mit den Eingabeparametern RelativeChangeOfZoombar, firstZoombar, secondZoombar implementiert werden:

```
     private Tuple<IZoomablexAxis, zoombarRange> GetSyncronizedRan-
     ges(IzoomablexAxis zoomableXAxis1, iZoomableXAxis zoomablexAxis2)
         {
             if (zoomablexAxis1 == null ∥ zoomablexAxis2 == null) return
     null;
            ZoombarRange zoombar1Range = zoomableXAxis1.GetRange();
            ZoombarRange zoombar2Range = zoomablexAxis2.GetRange();
            var zoombarlRangeSpan = (zoombar1Range.Maximum - zoom-
    bar1Range.Minimum) * (zoomableXAxis1.XAxisEnd - zoo-
     mablexAxis1.XAxisStart);
            var zoombar2RangeSpan = (zoombar2Range.Maximum - zoom-
     bar2Range.Minimum) * (zoomablexAxis2.XAxisEnd - Zoo-
     mableXAxis2.XAxisXtart);
            if (Math.Round(zoombar1RangeSpan, 5) < Math.Round(zoom-
     bar2RangeSpan, 5))
            {
                if (zoombar1Range.Minimum < 1 - zoombar1Range.Maximum)
                    return new Tuple<IZoomableXAxis, zoombarRange>(zoo-
     mableXAxis2, new ZoombarRange { Minimum = zoombar2Range.Minimum, Maxi-
     mum = zoombar2Range.Minimum + RelativeChangeOfSecondZoombar(zoom-
     bar1Range.Maximum - zoombar1Range.Minimum, zoomableXAxis1, zoo-
     mableXAxis2) });
                else return new Tuple<IZoomableXAxis, ZoombarRange>(zoo-
     mablexAxis2, new ZoombarRange { Minimum = zoombar2Range.Maximum - Re-
     lativeChangeOfSecondZoombar(zoombar1Range.Maximum - zoombar1Range.Mi-
     nimum, zoomableXAxis1, zoomableXAxis2), Maximum = zoombarzRange.Maxi-
     mum });
            }
            else if (Math.Round(zoombarlRangespan, 5) > Math.Round(zoom-
     bar2Rangespan, 5))
            {
                if (zoombar2Range.Minimum < 1 - zoombar2Range.Maximum)
                    return new Tuple<IZoomableXAxis, zoombarRange>(zoo-
    mableXAxis1, new ZoombarRange { Minimum = zoombar1Range.Minimum, Maxi-
    mum = zoombar1Range.Minimum + RelativeChangeOfsecondZoombar(zoom-
     bar2Range.Maximum - zoombar2Range.Minimum, zoomableXAxis2, zoo-
    mablexAxis1) });
                else return new Tuple<IZoomableXAxis, zoombarRange>(zoo-
    mableXAxisl, new ZoombarRange { Minimum = zoombar1Range.Maximum - Re-
     lativeChangeofSecondZoombar(zoombar2Range.Maximum - zoombar2Range.Mi-
     nimum, zoomableXAxis2, zoomableXAxis1), Maximum = zoombar1Range.Maxi-
    mum });
            }
            return null;
        }
```

Mit einer Methode RelativeChangeOfSecondZoombar 960 (Figur 9) kann die Berechnung des Range.Maximum der zweiten Zoombar folgendermaßen implementiert werden:

```
     RelafiveChangeOfSecondZoombar =
     relativeChangeOfFirstZoombar * (firstZoombar.XAxisEnd-firstZoom-
     bar.xAxisStart)/(secondzoombar.x,4xisEnd = secondzoombar.XAxisStart);
        private double RelativeChangeOfSecondZoombar(double rela-
     tiveChangeOfFirstZoombar, IZoomableXAxis firstZoombar, IZoomablexAxis
     secondZoombar)
        {
            return relativeChangeOfFirstZoombar * (firstZoombar.XAxisEnd -
    firstZoombar.XAxisStart) / (secondZoombar.XAxisEnd - secondzoom-
    bar.XAxisStart);
        }
```

**Figur 10** zeigt ein Prozessflussdiagramm, welches einen Prozess des Verlinkens der Zoombars 715, 720, 815, 820, wie weiter oben mit Bezug auf **Figuren 7** und **8** erläutert, visualisiert. Der Prozess wird mithilfe einer beispielhaften Implementierung der jeweiligen Methoden näher erläutert. Zunächst kann ein Benutzer mithilfe einer entsprechenden Aktivierung, z.B. durch Aktivierung des Buttons 735 in **Figur 7** bzw. 835 in **Figur 8**, im Folgenden auch "LinkTrendsToggle"-Button genannt, das Verlinken bzw. Entlinken zweier Zoombars 715, 720, 815, 820 starten 1005 (Link Zoombars). Beispielsweise kann der "LinkTrendsToggle"-Button per Binding an ein boolsches Property "AreTrendsLinked" gebunden sein, wie folgender beispielhafter Implementierung zu entnehmen ist:

```
    <ToggleButton x:Name="LinkTrendsToggleButton"
          width="40"
          Height="39"
          IsChecked="{Binding AreTrendsLinked,
                     RelativeSource={Relativesource TemplatedParent}}"
           Style="{DynamicResource TrendDisplay_LinkButtonStyle}"
           self:FocusAttachments.SetFocusable="True"
           IsTabStop="True"
           ToolTip="{Binding Ischecked, RelativeSource={RelativeSource
     Self}, Converter={converters:BoolToStringergConverter False= {x:Static
     resources:Resource.UnLinkedButtonToolTip}, True= {x:Static
     resources:Resource.LinkedButtonToolTip}}}"
                                                     />
     public bool AreTrendsLinked
       {
           get
           {
              return (bool)Getvalue(AreTrendsLinkedProperty);
           }
           set
           {
              this.SetValue(AreTrendsLinkedProperty, value);
           }
       }.
```

Die boolsche Property "AreTrendsLinked", welche feststellen kann, ob die entsprechenden Trends bereits verlinkt sind, kann dabei an eine DependencyProperty "AreTrendsLinkedProperty" geknüpft werden, welche bei entsprechender Aktivierung des "LinkTrendsToggle"-Buttons eine Methode "OnAreTrendsLinked" 1010 aufrufen kann, wie folgender beispielhafter Implementierung zu entnehmen ist:

```
     public static readonly DependencyProperty AreTrendsLinkedProperty =
     DependencyProperty.Register(
            "AreTrendsLinked", typeof(bool), typeof(TrendDisplay), new
     FrameworkPropertyMetadata(false, OnAreTrendsLinked));
     private static void OnAreTrendsLinked(DependencyObject d,
     DependencyPropertyChangedEventArgs e)
        {
            var areTrendsLinked = (bool)e.NewVAlue;
            var trendDisplay = d as TrendDisplay;
            if (trendDisplay == null)
            {
                return;
            }
            if (areTrendsLinked)
        {
    trendDisplay.zoombarManager.LinkZoombars(trendDispLay.trendControl1.z
    oomBar, trendDisplay.trendControl2.ZoomBar);
         }
         else
         {
             trendDisplay.zoombarManager.UnlinkZoombars();
                if (trendDisplay.trendControl1.Isvisible)
                {
                    (trendDisplay.trendControll.ExpandZoombarToMaxCommand
     as SAFCommand).RaiseCanExecuteChanged();
                }
                if (trendDisplay.trendControl2.Isvisible)
                {
                    (trendDisplay.trendControl2.ExpandZoombarToMaxCommand
     as SAFCommand).RaiseCanExecuteChanged();
                }
             }
         }.
```

Sind die Trends bereits verlinkt, kann die Methode "OnAreTrendsLinked" die Methode "UnlinkZoombars" 1020 aufrufen. Sind die Trends nicht verlinkt, kann die Methode "OnAreTrendsLinked" entsprechend die Methode "LinkZoombars" 1035 aufrufen. Dabei kann der boolsche Zustand der boolschen Property "AreTrendsLinked" entsprechend geändert werden. Es folgt eine beispielhafte Implementierung der Methoden "LinkZoombars" und "UnlinkZoombars":

```
         public void LinkZoombars(IZoomableXAxis zoomablexAxis1,
     IZoomableXAxis zoomableXAxis2)
         {
            if (trendsAreLinked == true) return;
            this.zoomables = new Dictionary<int, IzoomablexAxis>();
            this.zoomables.Add(1, zoomableXAxis1);
            this.zoomables.Add(2, zoomableXAxis2);
            // if range of the one zoombar changes: call OnRangeChanged
     on the other (and prevent further zoombarChangedNotification)
            this.linkSubscriptionTOken =
    this.zoombarChangedNotification.Subscribe(PublishActionLink);
            trendsAreLinked = true;
        }
         public void unlinkzoombars()
         {
            if(this.zoomables != null) this.zoomables.Clear();
            if(linkSubscriptionTOken != null)
     this.zoombarChangedNotification.Unsubscribe(this.linkSubscriptionToke
     n) ;
            this.linkSubscriptionToken = null;
            trendsAreLinked = false;
         }
```

Beim Aufrufen der Methode "LinkZoombars" 1035 kann ein Event "OnZoombarChangedNotification" abonniert 1040 (ZoombarChangedNotificationSubscribe) und mit einer Methode "PublishActionLink" 1045 verknüpft werden 1065 (Zoombars are linked). Das Event "OnZoombarChangedNotification" und folglich die Methode "PublishAction-Link" kann bei jedem Verschieben eines Schiebereglers entlang jeder Zoombar aufgerufen werden. In der Methode "PublishActionLink" kann die Funktionalität, dass ein Benutzer beide Zeitfenster mithilfe eines einzigen Schiebereglers steuern kann, implementiert sein. Mithilfe dieser Funktionalität kann der Benutzer beide Zeitfenster steuern, indem er den Schieberegler über die Zoombar einer Einheit (Aktor) verschiebt 1050 (ZoombarPositionChanged), wodurch das Zeitfenster entsprechend verschoben wird. Das Verschieben des Zeitfensters kann dabei sowohl auf die Messdaten des Aktors 1055 (ChangeFirstZoombar) als auch auf die Messdaten der zweiten Einheit (Reaktor) 1060 (ChangeSecondZoombar) angewandt werden, soweit dies für beide Einheiten möglich ist (wie weiter oben mit Bezug auf Figur 6 beschrieben). Es folgt eine beispielhafte Implementierung der Methode "PublishActionLink":

```
     private void PublishActionLink(ZoombarRangeChangedNotificationArgs
     args)
         {
            // if trends are not linked: nothing to do
            if (!this.trendsAreLinked) return;
            // all range changed call the notification again, then: do
     nothing
            if (inLinkAction ∥ inSyncAction ∥ inExpandToMaxAction)
     return;
             inLinkAction = true;
             var otherzoomable = this.zoomables.First(z => z.value ==
     args.zoomablexAxis);
             var key = otherzoomable.Key;
             if (key == 1)
             {
                // correct the second zoombar
                this.OnRangeChanged(args.Args.NewRange,
     args.Args.OldRange, this.zoomables[1], this.zoomables[2]);
             }
             else
             {
                // correct the first zoombar
                this.OnRangechanged(args.Args.NewRange,
     args.Args.Oldrtange, this.zoomables[2], this.zoomables[1]);
             }
             inLinkACtion = false;
         }
```

Beim Aufruf der Methode "UnlinkZoombars" 1020 kann ein Abbonement zum Event "OnZoombarChangedNotification" wieder gekündigt werden 1025 (ZoombarChanged-NotificationUnSubscribe), so dass sich ein Verschieben eines Zeitfensters nicht mehr auf das andere Zeitfenster auswirkt 1030 (Zoombars are unlinked).

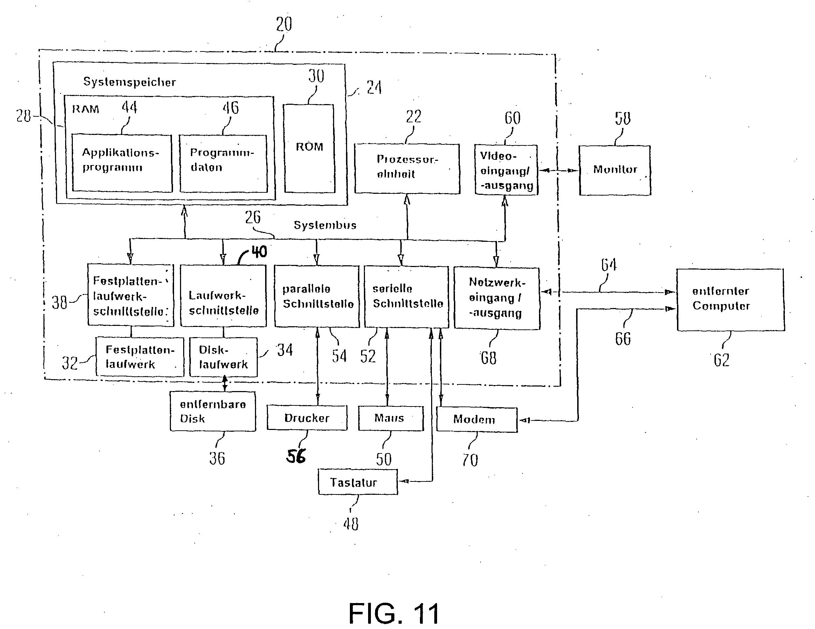

Bezugnehmend auf **Figur 11** wird ein beispielhaftes System zum Implementieren der Erfindung beschrieben. Ein beispielhaftes System umfasst eine universelle Rechnereinrichtung in der Form einer herkömmlichen Rechnerumgebung 20 z.B. ein "personal computer" (PC) 20, mit einer Prozessoreinheit 22, einem Systemspeicher 24 und einem Systembus 26, welcher eine Vielzahl von Systemkomponenten, unter anderem den Systemspeicher 24 und die Prozessoreinheit 22 verbindet. Die Prozessoreinheit 22 kann arithmetische, logische und/oder Kontrolloperationen durchführen, indem auf den Systemspeicher 24 zugegriffen wird. Der Systemspeicher 24 kann Informationen und/oder Instruktionen zur Verwendung in Kombination mit der Prozessoreinheit 22 speichern. Der Systemspeicher 24 kann flüchtige und nichtflüchtige Speicher, beispielsweise "random access memory" (RAM) 28 und "Nur-Lesespeicher" (ROM) 30 beinhalten. Ein Grund-Eingabe-Ausgabe-System (BIOS), das die grundlegenden Routinen enthält, welche helfen, Informationen zwischen den Elementen innerhalb des PCs 20, beispielsweise während des Hochfahrens, zu transferieren, kann in dem ROM 30 gespeichert sein. Der Systembus 26 kann eine von vielen Busstrukturen sein, unter anderem ein Speicherbus oder ein Speichercontroller, ein peripherer Bus und ein lokaler Bus, welcher eine bestimmte Busarchitektur aus einer Vielzahl von Busarchitekturen verwendet.

Der PC 20 kann weiterhin ein Festplattenlaufwerk 32 zum Lesen oder Schreiben einer Festplatte (nicht gezeigt) aufweisen und ein externes Disklaufwerk 34 zum Lesen oder Schreiben einer entfernbaren Disk 36 bzw. eines entfernbaren Datenträgers. Die entfernbare Disk kann eine magnetische Disk bzw. eine magnetische Diskette für ein magnetisches Disklaufwerk bzw. Diskettenlaufwerk oder eine optische Diskette, wie z.B. eine CD-ROM, für ein optisches Disklaufwerk sein. Das Festplattenlaufwerk 32 und das externe Disklaufwerk 34 sind jeweils mit dem Systembus 26 über eine Festplattenlaufwerkschnittstelle 38 und eine Disklaufwerkschnittstelle 40 verbunden. Die Laufwerke und die zugeordneten computerlesbaren Medien stellen einen nichtflüchtigen Speicher computerlesbarer Instruktionen, Datenstrukturen, Programm-Modulen und anderer Daten für den PC 20 zur Verfügung. Die Datenstrukturen können die relevanten Daten zum Implementieren eines wie oben beschriebenen Verfahrens aufweisen. Obwohl die beispielshaft beschriebene Umgebung eine Festplatte (nicht gezeigt) und eine externe Disk 42 verwendet, ist für den Fachmann offensichtlich, dass andere Typen computerlesbarer Medien, welche computerzugreifbare Daten speichern können, in der beispielhaften Arbeitsumgebung verwendet werden können, wie z.B. magnetische Kassetten, Flash-Memory Karten, digitale Videodisketten, Random-Access-Speicher, Nur-Lesespeicher, usw.

Eine Vielzahl von Programm-Modulen, insbesondere ein Betriebssystem (nicht gezeigt) ein oder mehrere Applikationsprogramme 44 oder Programm-Module (nicht gezeigt) und Programmdaten 46, können auf der Festplatte, der externen Disk 42, dem ROM 30 oder dem RAM 28 gespeichert werden. Die Applikationsprogramme können zumindest einen Teil der Funktionalität, wie in **Figur 1** bzw. **Figuren 5 bis 9** gezeigt, umfassen.

Ein Benutzer kann Kommandos und Information, wie oben beschrieben, in den PC 20 anhand von Eingabevorrichtungen, wie z.B. einer Tastatur bzw. eines Keyboards 48 und einer Computermaus 50, eingeben. Andere Eingabevorrichtungen (nicht gezeigt) können ein Mikrofon und/andere Sensoren, einen Joystick, ein Spielpolster bzw. -kissen, einen Scanner oder ähnliches umfassen. Diese oder andere Eingabevorrichtungen können mit der Prozessoreinheit 22 anhand einer seriellen Schnittstelle 52 verbunden sein, welche mit dem Systembus 26 gekoppelt ist oder können anhand anderer Schnittstellen, wie z.B. einer parallelen Schnittstelle 54, eines Spieleports oder eines universellen seriellen Busses (USB), verbunden sein. Weiterhin kann Information mit einem Drucker 56 gedruckt werden. Der Drucker 56 und andere parallele Eingabe/Ausgabevorrichtungen können mit der Prozessoreinheit 22 durch die parallele Schnittstelle 54 verbunden sein. Ein Monitor 58 oder andere Arten von Anzeigevorrichtung(en) ist/sind mit dem Systembus 26 mittels einer Schnittstelle, wie z.B. eines Videoeingang/-ausgangs 60, verbunden. Zusätzlich zu dem Monitor kann die Rechnerumgebung 20 andere periphere Ausgabevorrichtungen (nicht gezeigt), wie z.B. Lautsprecher oder akustische Ausgänge, umfassen.

Die Rechnerumgebung 20 kann mit anderen elektronischen Vorrichtungen, z.B. einem Computer, einem Schnurtelefon, einem schnurlosen Telefon, einem persönlichen digitalen Assistenten (PDA), einem Fernseher oder ähnlichem, kommunizieren. Um zu kommunizieren, kann die Rechnerumgebung 20 in einer vernetzten Umgebung arbeiten, wobei Verbindungen zu einem oder mehreren elektronischen Vorrichtungen verwendet werden. **Figur 11** stellt die mit einem "remote computer" bzw. entfernten Computer 62 vernetzte Rechnerumgebung dar. Der entfernte Computer 62 kann eine andere Rechnerumgebung, wie z.B. ein Server, ein Router, ein Netzwerk-PC, eine gleichwertige bzw. "peer" Vorrichtung oder andere gewöhnliche Netzwerkknoten sein und kann viele oder alle der hinsichtlich der Rechnerumgebung 20 oben beschriebenen Elemente umfassen. Die logischen Verbindungen, wie sie in **Figur 11** dargestellt sind, umfassen ein "local area network" (LAN) 64 und ein "wide area network" (WAN) 66. Solche Netzwerkumgebungen sind alltäglich in Büros, firmenweiten Computernetzwerken, Intranetzen und dem Internet.

Wenn eine Rechnerumgebung 20 in einer LAN-Netzwerkumgebüng verwendet wird, kann die Rechnerumgebung 20 mit dem LAN 64 durch einen Netzwerkeingang/-ausgang 68 verbunden sein. Wenn die Rechnerumgebung 20 in einer WAN-Netzwerkumgebung verwendet wird, kann die Rechnerumgebung 20 ein Modem 70 oder andere Mittel zum Herstellen einer Kommunikation über das WAN 66 umfassen. Das Modem 70, welches intern und extern bezüglich der Rechnerumgebung 20 sein kann, ist mit dem Systembus 26 mittels der seriellen Schnittstelle 52 verbunden. In der Netzwerkumgebung können Programm-Module, welche relativ zu der Rechnerumgebung 20 dargestellt sind, oder Abschnitte davon in einer entfernten Speichereinrichtung gespeichert sein, welche an oder von einem entfernten Computer 62 zugreifbar bzw. systemeigen sind. Weiterhin können andere Daten, welche für das oben beschriebene Verfahren bzw. System relevant sind, auf oder von dem entfernten Computer 62 zugreifbar vorliegen.

### Bezugszeichenliste

- 20: Rechnerumgebung
- 22: Prozessoreinheit
- 24: Systemspeicher
- 26: Systembus
- 28: random access memory (RAM)
- 30: Nur-Lesespeicher (ROM)
- 32: Festplattenlaufwerk
- 34: Disklaufwerk
- 36: entfernbare Disk
- 38: Festplattenlaufwerkschnittstelle
- 40: Disklaufwerkschnittstelle
- 44: Applikationsprogramm
- 46: Programmdaten
- 48: Tastatur
- 50: Computermaus
- 52: serielle Schnittstelle
- 54: parallele Schnittstelle
- 56: Drucker
- 58: Monitor
- 60: Videoeingang/ -ausgang
- 62: entfernter Computer
- 64: "local area network" (LAN)
- 66: "wide area network" (WAN)
- 68: Netzwerkeingang/ -ausgang
- 70: Modem
- 100: Anzeigeeinheit
- 105: Anzeigebereich
- 110: Anzeigebereich
- 115: Zoombar
- 120: Zoombar
- 125: Schieberegler
- 130: Schieberegler
- 135A-N: Einheit
- 140A-N: Sensor
- 145: Netzwerk (LAN, WAN)
- 150: Erfassungseinheit
- 155: Steuerungsmodul
- 160: Computersystem zur Steuerung, Erfassung, Regelung und/oder Analyse von biologischen, biochemischen, chemischen und/oder physikalischen Prozessen
- 200: Einheit
- 205: Sensor Device
- 210: Reaktor Device
- 215: Einzelsteuereinheit Device
- 220: Sensor, z.B. O₂/CO₂ Analysator bzw. Analysesensor
- 225: Bioreaktor
- 230: Einzelsteuereinheit, z.B. Waage
- 235: interne Einzelsteuereinheiten
- 305: Kommunikationssicht
- 310: Einrichtung
- 315: Anwender/Monitoring Sicht
- 320: Einheit
- 325: Kontrollmodul
- 330: Kontrollmodulvariable
- 335: Messdaten
- 340: Historical Batch Data Sicht
- 345: Batch
- 350: Start- und Stoppzeit
- 355: Einheit gespeichert in Batch
- 360: Kontrollmodul gespeichert in Batch
- 365: Datensatz Eigenschaft Sensor
- 370: Messdaten gespeichert in Batch
- 400: Gruppe
- 405A...N: Einheit
- 406A...N: Sensor Device
- 410A...N: Reaktor Device
- 415A...N: Einzelsteuereinheit Device
- 420A...N: Sensoren
- 425A...N: Bioreaktor
- 430A...N: Einzelsteuereinheit
- 435A...N: Interne Einzelsteuereinheiten
- 500: Exemplarische Anzeigeeinheit
- 505: Erster Anzeigebereich zur Darstellung von Messdaten
- 510: Zweiter Anzeigebereich zur Darstellung von Messdaten
- 515: Erste Zoombar
- 520: Zweite Zoombar
- 525: Erster Schieberegler
- 530: Zweiter Schieberegler
- 535: Button Verlinkung
- 540: Button Synchronisation
- 600: Exemplarische Anzeigeeinheit
- 605: Erster Anzeigebereich zur Darstellung von Messdaten
- 610: Zweiter Anzeigebereich zur Darstellung von Messdaten
- 615: Erste Zoombar
- 620: Zweite Zoombar
- 625: Erster Schieberegler
- 630: Zweiter Schieberegler
- 635: Button Verlinkung
- 640: Button Synchronisation
- 700: Exemplarische Anzeigeeinheit
- 705: Erster Anzeigebereich zur Darstellung von Messdaten
- 710: Zweiter Anzeigebereich zur Darstellung von Messdaten
- 715: Erste Zoombar
- 720: Zweite Zoombar
- 725: Erster Schieberegler
- 730: Zweiter Schieberegler
- 735: Button Verlinkung
- 740: Button Synchronisation
- 800: Exemplarische Anzeigeeinheit
- 805: Erster Anzeigebereich zur Darstellung von Messdaten
- 810: Zweiter Anzeigebereich zur Darstellung von Messdaten
- 815: Erste Zoombar
- 820: Zweite Zoombar
- 825: Erster Schieberegler
- 830: Zweiter Schieberegler
- 835: Button Verlinkung
- 840: Button Synchronisation
- 905: Synchronize Zoombars
- 910: OnCanSynchronize Trends
- 915: CanSynchronize Trends
- 920: CanNotSynchronize
- 925: CanSynchronize
- 930: Synchronize Button enabled
- 935: OnSynchronizeTrends
- 940: SyncZoombars
- 945: GetSynchronizedRange
- 950: Ranges Fit
- 955: Ranges Do Not Fit
- 960: RelativeChange of Second Zoombar
- 965: SetRelativeRange
- 970: Zoombars Have Same Range
- 1005: Unlink Zoombars
- 1010: OnAreTrendsLinked
- 1015: Trends are linked?
- 1020: Unlink Zoombars
- 1025: ZoombarChanged NotificationUnSubscribe
- 1030: Zoombars are unlinked
- 1035: Link Zoombars
- 1040: ZoombarChanged NotificationSubscribe
- 1045: Publish ActionLink
- 1050: Zoombar Position Changed
- 1055: ChangeFirstZoombar
- 1060: ChangeSecondZoombar
- 1065: Zoombars are linked

## Patentansprüche

1. Computersystem (160) zur Steuerung, Erfassung, Regelung und/oder Analyse von biologischen, biochemischen, chemischen und/oder physikalischen Prozessen in Bioreaktoren und/oder chemischen Reaktoren, umfassend:
zumindest zwei Einheiten (135 A-N, 200, 320, 405A-N), wobei jede Einheit zumindest einen Bioreaktor oder einen chemischen Reaktor umfasst, welcher eingerichtet ist, einen Stoff aufzunehmen, um an diesem Stoff zumindest einen biologischen, biochemischen, chemischen und/oder physikalischen Prozess durchzuführen;
wobei jede der Einheiten (135 A-N, 200, 320, 405A-N) zumindest einen Sensor (140A-N, 220, 420A-N) aufweist, welcher dem jeweiligen Bioreaktor oder chemischen Reaktor zugeordnet ist und welcher eingerichtet ist, Messdaten (335) betreffend den biologischen, biochemischen, chemischen und/oder physikalischen Prozess zu erfassen;
zumindest eine Anzeigeeinheit (100, 500, 600, 700, 800), über welche die erfassten Messdaten (335) der Prozesse der zwei Einheiten (135 A-N, 200, 320, 405A-N) jeweils in einer zeitlichen Korrelation dargestellt werden, welche Aufschlüsse über einen Zusammenhang der dargestellten Messdaten (335) ermöglicht; und **gekennzeichnet durch**:
eine Erfassungseinheit (150), welche eingerichtet ist,
- die zeitliche Korrelation zu erkennen, indem sie einen Verlauf der Messdaten (335) der jeweiligen Einheiten (135 A-N, 200, 320, 405A-N) miteinander vergleicht; und
- eine Anzeige von einander bzw. sich entsprechenden Messdaten (335) der jeweiligen Einheiten (135 A-N, 200, 320, 405A-N) unabhängig von einer absoluten Zeit ihrer Erfassung über die Anzeigeeinheit (100, 500, 600, 700, 800) zu ermöglichen.

2. Computersystem nach Anspruch 1, wobei die zeitliche Korrelation dargestellt werden kann, indem ein Zeitfenster spezifischer Größe derart über die Messdaten (335) der jeweiligen Einheiten (135 A-N, 200, 320, 405A-N) angewandt wird, dass Messdaten (335), die sich im Zeitfenster befinden, unabhängig von einer absoluten Zeit ihrer Erfassung zeitlich korreliert dargestellt werden.

3. Computersystem nach einem der vorangehenden Ansprüche, wobei die zeitliche Korrelation in Abhängigkeit einer Eingabe eines Benutzers eingestellt werden kann.

4. Computersystem nach Anspruch 3, wobei die Messdaten (335) der zwei Einheiten (135 A-N, 200, 320, 405A-N) über die Anzeigeeinheit (100, 500, 600, 700, 800) in jeweils einem Anzeigebereich (105, 110, 505, 510, 605, 610, 705, 710, 805, 810) angezeigt werden;
wobei die Einstellung zudem beinhalten kann, dass der Benutzer in jedem Anzeigebereich (105, 110, 505, 510, 605, 610, 705, 710, 805, 810) ein Zeitfenster jeweils an einen spezifischen Zeitpunkt der Messdaten (335) mithilfe eines Schiebereglers (825, 830), welcher entlang der Messdaten (335) im jeweiligen Anzeigebereich (105, 110, 505, 510, 605, 610, 705, 710, 805, 810) verschoben werden kann, anwendet.

5. Computersystem nach einem der vorangehenden Ansprüche, zusätzlich umfassend:
ein Steuerungsmodul (155), welches eingerichtet ist, zumindest zwei Einheiten (135 A-N, 200, 320, 405A-N) zu einer Gruppe zusammenzufassen.

6. Computersystem nach Anspruch 5, wobei das Steuerungsmodul (155) zudem eingerichtet ist, Zeitfenster, die über Messdaten (335) der jeweiligen Einheiten (135 A-N, 200, 320, 405A-N) angewandt sind, welche in jeweils einem Anzeigebereich (105, 110, 505, 510, 605, 610, 705, 710, 805, 810) angezeigt werden, miteinander zu verlinken, so dass ein Benutzer die Zeitfenster mithilfe eines Schiebereglers (825, 830) steuern kann, indem er die Zeitfenster synchron über die Messdaten (335) der jeweiligen Einheiten (135 A-N, 200, 320, 405A-N) verschiebt.

7. Computersystem nach Anspruch 5, wobei das Steuerungsmodul (155) zudem eingerichtet ist, den Prozess, welcher an dem Stoff der jeweiligen Einheiten (135 A-N, 200, 320, 405A-N) durchgeführt werden soll, zum selben Zeitpunkt, unabhängig vom absoluten Zeitpunkt, zu starten.

8. Computer-implementiertes Verfahren zur Steuerung, Erfassung, Regelung und/oder Analyse von biologischen, biochemischen, chemischen und/oder physikalischen Prozessen in Bioreaktoren und/oder chemischen Reaktoren, umfassend:
Bereitstellen von zumindest zwei Einheiten (135 A-N, 200, 320, 405A-N), wobei jede Einheit zumindest einen Bioreaktor oder einen chemischen Reaktor umfasst, welcher eingerichtet ist, einen Stoff aufzunehmen, um an diesem Stoff zumindest einen biologischen, biochemischen, chemischen und/oder physikalischen Prozess durchzuführen, wobei jede der Einheiten (135 A-N, 200, 320, 405A-N) zumindest einen Sensor aufweist, welcher dem jeweiligen Bioreaktor oder chemischen Reaktor zugeordnet ist,
Erfassen von Messdaten (335), durch die Sensoren, betreffend den jeweiligen biologischen, biochemischen, chemischen und/oder physikalischen Prozess, und
Anzeigen der erfassten Messdaten (335) der Prozesse der zwei Einheiten (135 A-N, 200, 320, 405A-N) in einer zeitlichen Korrelation durch eine Anzeigeeinheit (100, 500, 600, 700, 800),
wobei die zeitliche Korrelation Aufschlüsse über einen Zusammenhang der dargestellten Messdaten (335) ermöglicht; **gekennzeichnet durch** die Schritte:
Erkennen der zeitlichen Korrelation **durch** eine Erfassungseinheit (150), indem ein Verlauf der Messdaten (335) der jeweiligen Einheiten (135 A-N, 200, 320, 405A-N) miteinander verglichen wird; und
Anzeigen von einander bzw. sich entsprechenden Messdaten (335) der jeweiligen Einheiten (135 A-N, 200, 320, 405A-N) unabhängig von einer absoluten Zeit ihrer Erfassung, **durch** die Anzeigeeinheit (100, 500, 600, 700, 800).

9. Verfahren nach Anspruch 8, wobei die zeitliche Korrelation dargestellt werden kann, indem ein Zeitfenster spezifischer Größe derart über die Messdaten (335) der jeweiligen Einheiten (135 A-N, 200, 320, 405A-N) angewandt wird, dass Messdaten (335), die sich im Zeitfenster befinden, unabhängig von einer absoluten Zeit ihrer Erfassung zeitlich korreliert dargestellt werden.

10. Verfahren nach einem der Ansprüche 8 oder 9, wobei die zeitliche Korrelation in Abhängigkeit einer Eingabe eines Benutzers eingestellt werden kann;
die Messdaten (335) der zwei Einheiten (135 A-N, 200, 320, 405A-N) über die Anzeigeeinheit (100, 500, 600, 700, 800) in jeweils einem Anzeigebereich (105, 110, 505, 510, 605, 610, 705, 710, 805, 810) angezeigt werden können; und
die Einstellung zudem beinhalten kann, dass der Benutzer in jedem Anzeigebereich (105, 110, 505, 510, 605, 610, 705, 710, 805, 810) ein Zeitfenster jeweils zu einem spezifischen Zeitpunkt der Messdaten (335) mithilfe eines Schiebereglers (825, 830), welcher entlang der Messdaten (335) im jeweiligen Anzeigebereich (105, 110, 505, 510, 605, 610, 705, 710, 805, 810) verschoben werden kann, anwendet.

11. Verfahren nach einem der Ansprüche 8 bis 10, zudem umfassend:
Zusammenfassen von zumindest zwei Einheiten (135 A-N, 200, 320, 405A-N) zu einer Gruppe, über ein Steuerungsmodul (155), so dass die Gruppe über das Steuerungsmodul (155) gesteuert werden kann;
wobei das Verfahren zudem umfassen kann:
Verlinken von Zeitfenstern, die über Messdaten (335) der jeweiligen Einheiten (135 A-N, 200, 320, 405A-N) angewandt sind und jeweils in einem Anzeigebereich (105, 110, 505, 510, 605, 610, 705, 710, 805, 810) angezeigt werden, über das Steuerungsmodul (155), so dass ein Benutzer die Zeitfenster mithilfe eines Schiebereglers (825, 830) steuern kann, indem er die Zeitfenster synchron über die Messdaten (335) der jeweiligen Einheiten (135 A-N, 200, 320, 405A-N) verschiebt.

12. Verfahren nach einem der Ansprüche 8 bis 11, zudem umfassend:
Starten der Prozesse, die an dem Stoff der jeweiligen Einheiten (135 A-N, 200, 320, 405A-N) durchgeführt werden sollen, zum selben Zeitpunkt, unabhängig vom absoluten Zeitpunkt, über das Steuerungsmodul (155).

13. Computerprogrammprodukt umfassend Programmteile, welche, wenn in einem Computer geladen, zur Durchführung eines computer-implementierten Verfahrens nach einem der Ansprüche 8 bis 12 ausgelegt sind.

14. Graphische Benutzerschnittstelle zur Steuerung, Erfassung, Regelung und/oder Analyse von biologischen, biochemischen, chemischen und/oder physikalischen Prozessen in Bioreaktoren und/oder chemischen Reaktoren, umfassend:
zumindest zwei Anzeigebereiche (105, 110, 505, 510, 605, 610, 705, 710, 805, 810) spezifischer Größe und spezifischer Position, wobei jeder Anzeigebereich (105, 110, 505, 510, 605, 610, 705, 710, 805, 810) einer Einheit (135 A-N, 200, 320, 405A-N) zugeordnet ist, wobei jede Einheit zumindest einen Bioreaktor oder einen chemischen Reaktor umfasst, welcher eingerichtet ist, einen Stoff aufzunehmen, um an diesem Stoff zumindest einen biologischen, biochemischen, chemischen und/oder physikalischen Prozess durchzuführen,
wobei jede der Einheiten (135 A-N, 200, 320, 405A-N) zumindest einen Sensor aufweist, welcher dem jeweiligen Bioreaktor oder chemischen Reaktor zugeordnet ist und welcher eingerichtet ist, Messdaten (335) über den biologischen, biochemischen, chemischen und/oder physikalischen Prozess zu erfassen,
wobei in jedem Anzeigebereich (105, 110, 505, 510, 605, 610, 705, 710, 805, 810) die erfassten Messdaten (335) der Prozesse der zwei Einheiten (135 A-N, 200, 320, 405A-N) jeweils in einer zeitlichen Korrelation dargestellt werden, welche Aufschlüsse über einen Zusammenhang der dargestellten Messdaten (335) ermöglichen; **dadurch gekennzeichnet, daß**
die zeitliche Korrelation erkannt wird, indem ein Verlauf der Messdaten (335) der jeweiligen Einheiten (135 A-N, 200, 320, 405A-N) miteinander verglichen werden; und
die sich entsprechenden Messdaten (335) der jeweiligen Einheiten (135 A-N, 200, 320, 405A-N) unabhängig von einer absoluten Zeit ihrer Erfassung über die Anzeigeeinheit (100, 500, 600, 700, 800) im jeweiligen Anz7eigebereich angezeigt werden.

## Claims

1. A computer system (160) for control, acquisition, regulation and/or analysis of biological, biochemical, chemical and/or physical processes in bioreactors and/or chemical reactors, comprising:
at least two units (135 A-N, 200, 320, 405-N), each unit comprising at least one bioreactor or one chemical reactor configured to receive a substance in order to conduct at least one biological, biochemical, chemical and/or physical process on this substance;
wherein each of the units (135 A-N, 200, 320, 405-N) includes at least one sensor (140A-N, 220, 420A-N) assigned to the respective bioreactor or chemical reactor and configured to collect measurement data (335) relating to the biological, biochemical, chemical and/or physical process;
at least one display unit (100, 500, 600, 700, 800) via which the collected measurement data (335) of the processes of the two units (135 A-N, 200, 320, 405-N) is presented in temporal correlation, which allows obtaining information about a relation of the presented measurement data (335); and
**characterized by**:
an acquisition unit (150) configured to:
- identify the temporal correlation by comparing a course of the measurement data (335) of the respective units (135 A-N, 200, 320, 405-N) to each other, and
- allow displaying mutually corresponding measurement data (335) of the respective units (135 A-N, 200, 320, 405-N) independent of an absolute time of their collection via the display unit (100, 500, 600, 700, 800).

2. The computer system according to claim 1, wherein the temporal correlation can be presented by applying a time window of a specific size over the measurement data (335) of the respective units (135 A-N, 200, 320, 405-N) such that measurement data (335) present in the time window can be presented in a temporally correlated fashion independent of an absolute time of their collection.

3. The computer system according to one of the preceding claims, wherein the temporal correlation can be set depending on a user input.

4. The computer system according to claim 3, wherein the measurement data (335) of the two units (135 A-N, 200, 320, 405-N) is displayed via the display unit (100, 500, 600, 700, 800) in one display region (105, 110, 505, 510, 605, 610, 705, 710, 805, 810) each;
wherein the setting may further include that the user, in each display region (105, 110, 505, 510, 605, 610, 705, 710, 805, 810), applies a time window at respectively one specific time of the measurement data (335) by means of a scroll bar (825, 830), which can be shifted along the measurement data (335) in the respective display region (105, 110, 505, 510, 605, 610, 705, 710, 805, 810).

5. The computer system according to one of the preceding claims, additionally comprising:
a control module (155) configured to group at least two units (135 A-N, 200, 320, 405-N) together.

6. The computer system according to 5, wherein the control module (155) is further configured to link time windows applied over measurement data (335) of the respective units (135 A-N, 200, 320, 405-N), which are displayed in one display region (105, 110, 505, 510, 605, 610, 705, 710, 805, 810) each, to one another, so that a user can control the time windows by means of a scroll bar (825, 830) by shifting the time windows synchronously over the measurement data (335) of the respective units (135 A-N, 200, 320, 405-N).

7. The computer system according to claim 5, wherein the control module (155) is further configured to start the process to be conducted on the substance of the respective units (135 A-N, 200, 320, 405-N) at the same time independent of the absolute time.

8. A computer-implemented method for control, acquisition, regulation and/or analysis of biological, biochemical, chemical and/or physical processes in bioreactors and/or chemical reactors, comprising:
providing at least two units (135 A-N, 200, 320, 405-N), each unit comprising at least one bioreactor or one chemical reactor configured to receive a substance in order to conduct at least one biological, biochemical, chemical and/or physical process on this substance, wherein each of the units (135 AN, 200, 320, 405-N) includes at least one sensor assigned to the respective bioreactor or chemical reactor,
collecting, by the sensors, measurement data (335) relating to the biological, biochemical, chemical and/or physical process, and
displaying the collected measurement data (335) of the processes of the two units (135 A-N, 200, 320, 405-N) in temporal correlation by a display unit (100, 500, 600, 700, 800),
wherein the temporal correlation allows obtaining information about a relation of the presented measurement data (335);
**characterized by** the steps of:
identifying the temporal correlation an acquisition unit (150) by comparing a course of the measurement data (335) of the respective units (135 A-N, 200, 320, 405-N) to each other; and
displaying mutually corresponding measurement data (335) of the respective units (135 A-N, 200, 320, 405-N) independent of an absolute time of their collection by the display unit (100, 500, 600, 700, 800).

9. The method according to claim 8, wherein the temporal correlation can be presented by applying a time window of a specific size over the measurement data (335) of the respective units (135 A-N, 200, 320, 405-N) such that measurement data (335) present in the time window can be presented in a temporally correlated fashion independent of an absolute time of their collection.

10. The method according to one of claims 8 or 9, wherein the temporal correlation can be set depending on a user input;
the measurement data (335) of the two units (135 A-N, 200, 320, 405-N) can be displayed via the display unit (100, 500, 600, 700, 800) in one display region (105, 110, 505, 510, 605, 610, 705, 710, 805, 810) each; and
the setting may further include that the user, in each display region (105, 110, 505, 510, 605, 610, 705, 710, 805, 810), applies a time window at respectively one specific time of the measurement data (335) by means of a scroll bar (825, 830), which can be shifted along the measurement data (335) in the respective display region (105, 110, 505, 510, 605, 610, 705, 710, 805, 810).

11. The method according to one of claims 8 to 10, further comprising:
grouping at least two units (135 A-N, 200, 320, 405-N) together by a control module (155), so that the group can be controlled by the control module (155);
wherein the method can further comprise:
linking time windows applied over measurement data (335) of the respective units (135 A-N, 200, 320, 405-N) and displayed in one display region (105, 110, 505, 510, 605, 610, 705, 710, 805, 810) each to one another, by the control module (155), so that a user can control the time windows by means of a scroll bar (825, 830) by shifting the time windows synchronously over the measurement data (335) of the respective units (135 A-N, 200, 320, 405-N).

12. The method according to one of claims 8 to 11, further comprising:
starting the processes to be conducted on the substance of the respective units (135 A-N, 200, 320, 405-N) at the same time independent of the absolute time by the control module (155).

13. A computer program product comprising program parts, which, when loaded in a computer, are configured to perform a computer-implemented method according to one of claims 8 to 12.

14. A graphical user interface for control, acquisition, regulation and/or analysis of biological, biochemical, chemical and/or physical processes in bioreactors and/or chemical reactors, comprising:
at least two display regions (105, 110, 505, 510, 605, 610, 705, 710, 805, 810) of a specific size and specific position, each display region (105, 110, 505, 510, 605, 610, 705, 710, 805, 810) being assigned to a units (135 A-N, 200, 320, 405-N), each unit comprising at least one bioreactor or one chemical reactor configured to receive a substance in order to conduct at least one biological, biochemical, chemical and/or physical process on this substance,
wherein each of the units (135 A-N, 200, 320, 405-N) includes at least one sensor assigned to the respective bioreactor or chemical reactor and configured to collect measurement data (335) on the biological, biochemical, chemical and/or physical process,
wherein in each display region (105, 110, 505, 510, 605, 610, 705, 710, 805, 810) the collected measurement data (335) of the processes of the two units (135 A-N, 200, 320, 405-N) is presented in temporal correlation, which allow obtaining information about a relation of the presented measurement data (335);
**characterized in that**:
the temporal correlation is identified by comparing a course of the measurement data (335) of the respective units (135 A-N, 200, 320, 405-N) to each other, and
the mutually corresponding measurement data (335) of the respective units (135 A-N, 200, 320, 405-N) is displayed independent of an absolute time of their collection via the display unit (100, 500, 600, 700, 800) in the respective display region.

## Revendications

1. Système d'ordinateur (160) pour la commande, l'acquisition, la configuration et/ou l'analyse de processus biologiques, biochimiques, chimiques et/ou physiques dans des bioréacteurs et/ou des réacteurs chimiques, comportant:
au moins deux unités (135 A-N, 200, 320, 405 A-N),comportant chacune au moins un bioréacteur ou un réacteur chimique, qui est agencé pour recevoir une matiète, afin d'y effectuer au moins un processus biologique, biochimique, chimique et/ou physique ;
chacune des unités (135 A-N, 200, 320, 405 A-N) comportant au moins un senseur (140 A-N, 220, 420 A-N) associé au dit bioréacteur ou réacteur chimique et agencé pour acquérir des données de mesure (335) relatives au processus biologique, biochimique, chimique et/ou physique ;
au moins une unité d'affichage (100, 500, 600, 700, 800) qui représente les données de mesure (335) des processus de chacune des deux unités (135 A-N, 200, 320, 405 AN) en une corrélation chronologique qui permet des révélations concernant une relation entre les données de mesure (335) représentées, et
**caractérisé par**
une unité d'acquisition (150) agencée pour
- reconnaître la corrélation chronologique en comparant entre elles les évolutions des données de mesure (335) de chacune des unités (135 A-N, 200, 320, 405 AN);et
- permettre un affichage via l'unité d'affichage (100, 500, 600, 700, 800) de données de mesure correspondant l'une à l'autre (335) en provenance de chacune des unités (135 A-N, 200, 320, 405 A-N), indépendamment d'un temps d'acquisition absolu.

2. Système d'ordinateur selon la revendication 1, dans lequel la corrélation chronologique est représentable moyennant une fenêtre de temps de dimension spécifique appliquée aux données de mesure (335) de chacune des unités (135 A-N, 200, 320, 405 A-N) de telle sorte que les données de mesure (335) se trouvant dans la fenêtre de temps sont représentées en corrélation chronologique indépendamment d'un temps d'acquisition absolu.

3. Système d'ordinateur selon l'une des revendications précédentes dans lequel la corrélation chronologique est réglable en fonction de données introduites par un utilisateur.

4. Système d'ordinateur selon la revendication 3 dans lequel les données de mesure (335) des deux unités (135 A-N, 200, 320, 405 A-N) sont affichées par l'unité d'affichage (100, 500, 600, 700, 800) chaque fois dans un domaine d'affichage (105, 110, 505, 510, 605, 610, 705, 710, 805, 810) ; le réglage pouvant encore comporter l'application par l'utilisateur, dans chaque domaine d'affichage (105, 110, 505, 510, 605, 610, 705, 710, 805, 810), d'une fenêtre de temps chaque fois à un moment spécifique des données de mesure (335), moyennant un curseur (825, 830) qui est déplaçable le long des données de mesure (335) dans chacun des domaines d'affichage (105, 110, 505, 510, 605, 610, 705, 710, 805, 810).

5. Système d'ordinateur selon l'une des revendications précédentes comportant en plus un module de commande (155) agencé pour réunir au moins deux unités (135 A-N, 200, 320, 405 A-N) en un groupe.

6. Système d'ordinateur selon la revendication 5 dans lequel le module de commande (155) est agencé de manière à relier entre elles les fenêtres de temps qui sont appliquées sur les données de mesure (335) de chacune des unités (135 A-N, 200, 320, 405 A-N) et affichées chaque fois dans un domaine d'affichage (105, 110, 505, 510, 605, 610, 705, 710, 805, 810) de telle sorte qu'un utilisateur peut commander les fenêtres de temps moyennant un curseur (825, 830) en les déplaçant de façon synchrone sur les données de mesure (335) de chacune des unités (135 A-N, 200, 320, 405 A-N).

7. Système d'ordinateur selon la revendication 5, dans lequel le module de commande (155) est agencé de manière à démarrer le processus à effectuer sur la matière de chacune des unités (135 A-N, 200, 320, 405 A-N) au même moment, indépendamment du moment absolu.

8. Procédé implémenté par ordinateur pour la commande, l'acquisition, la configuration et/ou l'analyse de processus biologiques, biochimiques, chimiques et/ou physiques dans des bioréacteurs et/ou des réacteurs chimiques, comportant:
la mise à disposition d'au moins deux unités (135 A-N, 200, 320, 405 A-N) comportant chacune au moins un bioréacteur ou un réacteur chimique, qui est agencé pour recevoir une matière, afin d'y effectuer au moins un processus biologique, biochimique, chimique et/ou physique, chacune des unités (135 A-N, 200, 320, 405 AN) comportant au moins un senseur (140 A-N, 220, 420 A-N) associé au dit bioréacteur ou réacteur chimique,
l'acquisition par les senseurs de données de mesure (335) concernant chacun des processus biologiques, biochimiques, chimiques et/ou physiques, et
l'affichage par une unité d'affichage (100, 500, 600, 700, 800) des données de mesure acquises (335) des processus des deux unités (135 A-N, 200, 320, 405 A-N), dans une corrélation chronologique,
ladite corrélation chronologique permettant des révélations concernant une relation entre les données de mesure (335) représentées ;
**caractérisé par** les étapes :
reconnaissance de la corrélation chronologique par une unité d'acquisition (150), par comparaison des entre elles des évolutions des données de mesure (335) de chacune des unités (135 A-N, 200, 320, 405 A-N) ; et
affichage via l'unité d'affichage (100, 500, 600, 700, 800) de données de mesure correspondant l'une à l'autre (335) en provenance de chacune des unités (135 A-N, 200, 320, 405 A-N), indépendamment d'un temps d'acquisition absolu.

9. Procédé selon la revendication 8 dans lequel on peut représenter la corrélation chronologique en appliquant une fenêtre de temps de dimension spécifique aux données de mesure (335) de chacune des unités (135 A-N, 200, 320, 405 A-N) de telle sorte que les données de mesure (335) se trouvant dans la fenêtre de temps sont représentées en corrélation chronologique indépendamment d'un temps d'acquisition absolu.

10. Procédé selon l'une des revendications 8 ou 9 dans lequel on peut régler la corrélation chronologique en fonction de données introduites par un utilisateur ;
les données de mesure (335) des deux unité (135 A-N, 200, 320, 405 A-N) peuvent être affichées par l'unité d'affichage (100, 500, 600, 700, 800) chaque fois dans un domaine d'affichage (105, 110, 505, 510, 605, 610, 705, 710, 805, 810) ; et
le réglage peut encore comporter l'application par l'utilisateur, dans chaque domaine d'affichage (105, 110, 505, 510, 605, 610, 705, 710, 805, 810), d'une fenêtre de temps chaque fois à un moment spécifique des données de mesure (335) moyennant un curseur (825, 830) déplaçable le long des données de mesure (335) dans chacun des domaines d'affichage (105, 110, 505, 510, 605, 610, 705, 710, 805, 810).

11. Procédé selon l'une des revendications 8 à 10 comportant en plus :
rassemblement d'au moins deux unités (135 A-N, 200, 320, 405 A-N) en un groupe moyennant un module de commande (155) de telle sorte que le groupe est commandable par ledit module de commande (155) ;
le procédé comportant encore :
liaison entre elles des fenêtres de temps appliquées sur les données de mesure (335) de chacune des unités (135 A-N, 200, 320, 405 A-N) et affichées chaque fois dans un domaine d'affichage (105, 110, 505, 510, 605, 610, 705, 710, 805, 810), via le module de commande, de telle sorte qu'un utilisateur peut commander les fenêtres de temps moyennant un curseur (825, 830), en déplaçant les fenêtres de temps de manière synchrone sur les données de mesure (335) de chacune des unités (135 A-N, 200, 320, 405 A-N).

12. Procédé selon l'une des revendications 8 à 11 comportant en plus :
démarrage via le module de commande (155) des processus à effectuer sur la matière de chacune des unités (135 A-N, 200, 320, 405 A-N) au même moment, indépendamment du moment absolu.

13. Produit « programme d'ordinateur » comportant des parties de programme qui lorsqu'elles sont chargées dans un ordinateur sont agencées pour effectuer un procédé implémenté par ordinateur selon l'une des revendications 8 à 12.

14. Interface graphique pour la commande, l'acquisition, la configuration et/ou l'analyse de processus biologiques, biochimiques, chimiques et/ou physiques dans des bioréacteurs et/ou des réacteurs chimiques, comportant:
au moins deux domaines d'affichage (105, 110, 505, 510, 605, 610, 705, 710, 805, 810) de dimension spécifique et de position spécifique, chaque domaine d'affichage (105, 110, 505, 510, 605, 610, 705, 710, 805, 810) est associé à une unité (135 A-N, 200, 320, 405 A-N), chaque unité comportant au moins un bioréacteur ou un réacteur chimique agencé pour recevoir une matière afin d'y effectuer au moins un processus biologique, biochimique, chimique et/ou physique,
chaque unité (135 A-N, 200, 320, 405 A-N) comportant au moins un senseur associé au dit bioréacteur ou réacteur chimique et agencé pour acquérir des données de mesure (335) relatives au processus biologique, biochimique, chimique et/ou physique,
les données de mesure (335) acquises des processus des deux unités (135 A-N, 200, 320, 405 A-N) étant représentées dans chaque domaine d'affichage (105, 110, 505, 510, 605, 610, 705, 710, 805, 810) chaque fois dans une corrélation chronologique qui permet des révélations concernant une relation entre les données de mesure (335) représentées,
**caractérisée en ce**
**qu'**on reconnaît la corrélation chronologique par comparaison entre elles des évolutions des données de mesure (335) de chacune des unités (135 A-N, 200, 320, 405 A-N) ; et
**qu'**on affiche dans le domaine d'affichage correspondant, via l'unité d'affichage (100, 500, 600, 700, 800), les données de mesure (335) correspondant à chacune des unités (135 A-N, 200, 320, 405 A-N), indépendamment d'un temps d'acquisition absolu.
